(19) Europäisches Patentamt
European Patent Office
Office européen des brevets

(11) EP 3 626 820 A1

(12) **EUROPEAN PATENT APPLICATION**

(43) Date of publication:
**25.03.2020 Bulletin 2020/13**

(51) Int Cl.:
***C12N 15/113*** *(2010.01)*

(21) Application number: **18382676.7**

(22) Date of filing: **20.09.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Fundación Imdea Nanociencia**
**28049 Madrid (ES)**

(72) Inventors:
- **SOMOZA CALATRAVA, Álvaro**
  **28049 Madrid (ES)**
- **MILÁN ROIS, Paula**
  **28049 Madrid (ES)**
- **LATORRE LOZANO, Alfonso**
  **28029 Madrid (ES)**

(74) Representative: **ABG Intellectual Property Law, S.L.**
**Avenida de Burgos, 16D**
**Edificio Euromor**
**28036 Madrid (ES)**

# (54) ANTICANCER COMPOSITIONS CONTAINING MIRNA MIMICS AND USES THEREOF

(57) The invention relates to combinations comprising a miRNA-34a mimic, a miRNA-182 mimic, a miRNA-137 mimic and a miRNA-144 mimic as well as to synergistic compositions comprising said combination or miRNA mimics and a topoisomerase inhibitor. The combinations and compositions may also be provided as nanoparticles having the miRNA mimics and the topoisomerase inhibitor attached to the nanoparticle. The invention also provides the use of the combinations and compositions as defined above in the treatment of cancer and, more in particular, in the treatment of uveal melanoma.

EP 3 626 820 A1

SEQUENCE LISTING

<110> Fundación IMDEA Namociencia

<120> ANTICANCER COMPOSITION CONTAINING mirna MIMICS AND USES THEREOF

<130> P16587EP00

<160> 25

<170> PatentIn version 3.5

<210> 1
<211> 110
<212> RNA
<213> Artificial sequence

<220>
<223> miRNA-34a

<400> 1
ggccagcugu gaguguuucu uuggcagugu cuuagcuggu uguugugagc aauaguaagg 60

aagcaaucag caaguauacu gcccuagaag ugcugcacgu uguggggccc 110

<210> 2
<211> 110
<212> RNA
<213> Artificial sequence

<220>
<223> miRNA-182

<400> 2
gagcugcuug ccuccccccg uuuuuggcaa ugguagaacu cacacuggug agguaacagg 60

auccggugguu ucuagacuug ccaacuaugg ggcgaggacu cagccggcac 110

<210> 3
<211> 102
<212> RNA
<213> Artificial sequence

<220>
<223> miRNA-137

<400> 3
gguccucuga cucucuucgg ugacggguau ucuugggugg auaauacgga uuacguuguu 60

auugcuuaag aauacgcgua gucgaggaga guaccagcgg ca 102

<210> 4
<211> 86
<212> RNA
<213> Artificial sequence

<220>
<223> miRNA-144

<400> 4

uggggcccug gcugggauau caucauauac uguaaguuug cgaugagaca cuacaguaua 60

gaugauguac uaguccgggc accccc 86

<210> 5
<211> 22
<212> RNA
<213> Artificial sequence

<220>
<223> miRNA-34a (mature)

<400> 5
uggcaguguc uuagcugguu gu 22

<210> 6
<211> 24
<212> RNA
<213> Artificial sequence

<220>
<223> miRNA-182 (mature)

<400> 6
uuuggcaaug guagaacuca cacu 24

<210> 7
<211> 23
<212> RNA
<213> Artificial sequence

<220>
<223> miRNA-137 (mature)

<400> 7
uuauugcuua agaauacgcg uag 23

<210> 8
<211> 22
<212> RNA
<213> Artificial sequence

<220>
<223> miRNA-144 (mature)

<400> 8
ggauaucauc auauacugua ag 22

<210> 9
<211> 28
<212> DNA
<213> Artificial sequence

<220>
<223> DNA-34a

<400> 9
tggcagtgtc ttagctggtt gttaaaaa 28

<210> 10
<211> 29
<212> DNA
<213> Artificial sequence

<220>
<223> DNA-182

<400> 10
tttggcaatg gtagaactca cactaaaaa 29

<210> 11
<211> 28
<212> DNA
<213> Artificial sequence

<220>
<223> DNA-137

<400> 11
ttattgctta agaatacgcg tagaaaaa 28

<210> 12
<211> 27
<212> DNA
<213> Artificial sequence

<220>
<223> DNA-144

<400> 12
ggatatcatc atatactgta agaaaaa 27

<210> 13
<211> 28
<212> DNA
<213> Artificial sequence

<220>
<223> DNA-34a

<220>
<221> Thiol
<222> (28)..(28)

<400> 13
tggcagtgtc ttagctggtt gttaaaaa 28

<210> 14
<211> 29
<212> DNA
<213> Artificial sequence

<220>
<223> DNA-182

<220>
<221> Thiol
<222> (29)..(29)

<400> 14
tttggcaatg gtagaactca cactaaaaa 29

<210> 15
<211> 28
<212> DNA
<213> Artificial sequence

<220>
<223> DNA-137

<220>
<221> Thiol
<222> (28)..(28)

<400> 15
ttattgctta agaatacgcg tagaaaaa 28

<210> 16
<211> 27
<212> DNA
<213> Artificial sequence

<220>
<223> DNA-144

<220>
<221> Thiol
<222> (27)..(27)

<400> 16
ggatatcatc atatactgta agaaaaa 27

<210> 17
<211> 27
<212> RNA
<213> Artificial sequence

<220>
<223> RNA-34a pass

<220>
<221> Thiol
<222> (1)..(1)

<400> 17
aaaaaacaac cagcuaagac acugccu 27

<210> 18
<211> 22
<212> RNA

<213> Artificial sequence

<220>
<223> RNA-34a guide

<400> 18
uggcaguguc uuagcugguu gu 22

<210> 19
<211> 29
<212> RNA
<213> Artificial sequence

<220>
<223> RNA-182 pass

<220>
<221> Thiol
<222> (1)..(1)

<400> 19
aaaaaagugu gaguucuacc auugccaaa 29

<210> 20
<211> 24
<212> RNA
<213> Artificial sequence

<220>
<223> RNA-182 guide

<400> 20
uuuggcaaug guagaacuca cacu 24

<210> 21
<211> 28
<212> RNA
<213> Artificial sequence

<220>
<223> RNA-137 pass

<220>
<221> Thiol
<222> (1)..(1)

<400> 21
aaaaacuacg cguauucuua agcaauaa 28

<210> 22
<211> 23
<212> RNA
<213> Artificial sequence

<220>
<223> RNA-137 guide

<400> 22
uuauugcuua agaauacgcg uag 23

<210> 23
<211> 27
<212> RNA
<213> Artificial sequence

<220>
<223> RNA-144 pass

<220>
<221> Thiol
<222> (1)..(1)

<400> 23
aaaaacuuac aguauaugau gauaucc 27

<210> 24
<211> 22
<212> RNA
<213> Artificial sequence

<220>
<223> RNA-144 guide

<400> 24
ggauaucauc auauacugua ag 22

<210> 25
<211> 32
<212> DNA
<213> Artificial sequence

<220>
<223> AS1411 [25]

<220>
<221> dithiolane
<222> (32)..(32)

<400> 25
ggtggtggtg gttgtggtgg tggtggtttt tt 32

## Description

### TECHNICAL FIELD OF THE INVENTION

The present invention relates to the field of cancer therapeutics and, more in particular, to therapeutics using compounds that mimic the activity of endogenous miRNAs.

### BACKGROUND OF THE INVENTION

Uveal melanoma (UM) is a malignant intraocular tumor with a high metastatic risk. Unfortunately, the treatments for the metastatic disease are not effective, due to unique characteristics of this tumor, and also because the visible clinical features emerge at late stages of the disease. In the case of UM, there is no effective systemic therapy for metastatic UM, and drugs developed for other tumors, such as Irinotecan, which is used in colon and lung tumors, are used. Several chemotherapeutics agents are in clinical trials to treat UM, such as Selumetinib or AZD 8055. These agents are selective against MAPK and mTOR pathways respectively, however they are not very selective and healthy cells are also affected, therefore, more selective therapies are needed.

Therefore, novel approaches to tackle this disease are desirable.

### SUMMARY OF THE INVENTION

In a first aspect, the invention relates to a combination comprising a miRNA-34a mimic, a miRNA-182 mimic, a miRNA-137 mimic and a miRNA-144 mimic.

In a second aspect, the invention relates to the combination of the first aspect for use in medicine.

In another aspect, the invention relates to the combination of the first aspect for use in the treatment of cancer.

In another aspect, the invention relates to the combination of the first aspect for use as an adjuvant in the treatment of cancer with a chemotherapeutic compound.

### BRIEF DESCRIPTION OF THE FIGURES

**Figure 1: a-c** Cell viability assays in Mel 202 cells using different combination of oligonucleotides 1, 2, 3 and 4 ($p < 0.001$) (a) Mel 202 cells were transfected with individuals oligonucleotides (1, 2, 3 and 4) treatment at 140 nM/ well (b) Mel 202 cells were transfected with oligonucleotides in pairs (final oligonucleotide concentration = 140 nM). (c) Mel 202 cells were transfected with DNA mix 1 at 140 nM. d-e Immunofluorescence study in Mel 202 cells: (d) Representation of the fluorescence intensity (AU) of c-Met in cells untreated and treated with the DNA mix 1. (e) Representative immunofluorescence images of cells untreated (I) and treated with the DNA mix 1 (II). c-Met is shown in green and nucleus are marked in blue due to Hoechst staining. The DNA mix can reduce the expression of c-Met

**Figure 2:** (a) Cell viability assay using SN38 at different concentrations in Mel 202 cells. 100 nM is the IC50 in which the cell viability is reduced 50%. ($p < 0.001$) (b) Cell viability assay using SN38 and DNA mix 1 in Mel 202 cells. The viability is reduced 50% at very low concentration of SN38.

**Figure 3:** Flow cytometry graphs in Mel 202 cells. Green area: cells in G1 phase; Blue area: cells in S phase. Yellow area: cells in G2/M phase. (a) Untreated cells. (b) Cells treated with DNA mix. (c) Cells treated with SN38 (25 nM). (d) Cells treated with miRNA mimics and SN38.

**Figure 4:** AuNPs characterization (a-c) Hydrodynamic diameter size (nm) of the modified AuNPs and polydispersity index (PDI). (d) AuNPs spectrum where the corresponding plasmon band at 520 nm was noticeable.

**Figure 5:** (a) Alamar blue viability assay in MEL 202 of different AuNPs conditions (100 $\mu$l of AuNPs 287 DNA mix 2 were added in each well. The AuNPs SN38 wells were load with 25nM AuNP SN38. The 288 last conditions was load with 100 $\mu$l AuNP DNA mix 2 and 25 nM AuNP SN38. (b) Alamar blue 289 viability assay in MEL 202 of different AuNPs conditions (100 $\mu$l of AuNPs siRNA mix were add in 290 each well. The AuNPs SN38 wells were load with 25nM AuNP SN38. The last condition was load 291 with 100 $\mu$l AuNP siRNA mix and 25 nM AuNP SN38 ($p < 0.001$).

**Figure 6:** Flow cytometry graphs in Mel 202 cells. Green area: cells in G1 phase; Blue area: cells in S phase. Yellow area: cells in G2/M phase. (a) Untreated cells. (b) Cells treated with 100 $\mu$l AuNP DNA mix 2 (c) Cells treated with 100 $\mu$l AuNPs siRNA mix. (d) Cells treated with 25 nM AuNPs SN38 (e) Cells treated with 100 $\mu$l AuNPs DNA mix 2 and 25 nM AuNP SN38. (f) Cells treated with 100 $\mu$l AuNPs siRNA mix and 25 nM AuNP SN38.

## DETAILED DESCRIPTION OF THE INVENTION

Compositions comprising miRNA mimics

The authors of the present invention have identified that it is possible to reduce the viability of cells derived from uveal melanoma by treating the cells with a combination of nucleic acids that act as mimics of miRNA-34a, miRNA-182, miRNA-137 and miRNA-144

Accordingly, in a first aspect, the invention relates to a combination comprising a miRNA-34a mimic, a miRNA-182 mimic, a miRNA-137 mimic and a miRNA-144 mimic.

The term "combination" as used herein, refers to a composition comprising a plurality of components. The use of the term "combination" does not restrict the relative contents of the different components of the composition, which may be found in equimolar ratios, in the same weight ration or in different molar or weight ratios.

The term "miRNA mimic" as used herein refers to synthetic replicates of endogenous miRNAs which mimic the function or activity of a mature, endogenous miRNA thus increasing or replacing the intracellular function of said miRNA. miRNA mimics are capable of counteracting expression of the gene products the expression of which is down-regulated by the miRNA which they are mimicking.

The terms "microRNA" or "miRNA" or "miR" are used herein interchangeably to refer to a small non-coding RNA, which functions in transcriptional and/or post-transcriptional regulation of gene expression. In various embodiments, miRNAs have a hairpin structure comprising a duplex that is processed into a guide strand and a passenger strand.

"Pre-miRNA" or "pre-miR" means a non-coding RNA having a hairpin structure, which is the product of cleavage of a pri-miR by double-stranded RNA-specific ribonuclease.

"Pri-miRNA" or "pri-miR" means a non-coding RNA having a hairpin structure that is a substrate for double-stranded RNA-specific ribonuclease.

By the phrase "miRNA precursor" means a transcript that originates from a genomic DNA and that comprises a non-coding, structured RNA comprising one or more miRNA sequences. For example, in certain embodiments, a miRNA precursor is a pre-miRNA. In certain embodiments, a miRNA precursor is a pri-miRNA.

The miRNAs are referred to in the present invention by their standardized nomenclature, wherein:

- An uncapitalized "mir-" refers to a pre-miRNA, while a capitalized "miR-" refers to a mature form.
- miRNAs with nearly identical sequences are annotated with an additional lower case letter. For example, miR- 123a is closely related to miR- 123b.
- miRNAs that are 100 percent identical but are encoded at different places in the genome are indicated with additional dash-number suffix: miR-123-1 and miR-123-2 are identical but are produced from different pre-miRNAs.
- Species of origin is designated with a three-letter prefix, e.g., hsa-miR-123 would be from human (Homo sapiens) and oar-miR- 123 would be a sheep (Ovis aries) miRNA.
- When relative expression levels are known, an asterisk following the name indicates an miRNA expressed at low levels relative to the miRNA in the opposite arm of a hairpin. For example, miR-123 and miR-123* would share a pre-miRNA hairpin, but relatively more miR-123 would be found in the cell.
- The suffixes 5p and 3p indicate if a miRNA is derived from the 5'arm or the 3'arm of the pre-miRNA respectively. These suffices are used when both miRNAs are expressed at equal levels. For example miR423-5p and miR423-3p would share a pre-miRNA hairpin.

The nomenclature of the miRNA should not be given absolute weight as it is still subject to change. The sequence for the miRNAs for which mimicking compounds have been developed in the present invention is given in Table 1. According to the present invention, miRNAs as mentioned herein are capable of counteracting expression of specific gene products. As used herein, the term "miRNA" encompasses any isoform of the said miRNA and all members of the said miRNA family are capable of counteracting expression of the specific gene products. The term "miRNA" thus includes star-sequences and family members.

Typically, miRNA is double-stranded RNA which comprises a guide strand and a passenger strand, wherein either the guide strand, the passenger strand or both but preferably the guide strand contains a seed region.

As used herein, "guide strand" refers to a single-stranded nucleic acid molecule of an miRNA, which has a sequence sufficiently complementary to that of a target mRNA to hybridize to the target mRNA (e.g., in the 5' UTR, the coding region, or the 3' UTR) and to decrease or inhibit its translation. A passenger strand is also termed an "antisense strand."

As used herein, "passenger strand" refers to an oligonucleotide strand of an miRNA, which has a sequence that is complementary or substantially complementary to that of the guide strand. In some embodiments, the passenger strand may target an mRNA by hybridizing to the target mRNA (e.g., in the 5' UTR, the coding region, or the 3' UTR) and to decrease or inhibit its translation A passenger strand is also termed a "sense strand."

As used herein, "seed region" refers to the portion of an oligonucleotide strand that hybridizes to a target RNA (e.g. a miRNA), and involves a sequence that is complementary or substantially complementary to the target RNA. A seed region may be 6, 7, or 8 nucleotides in length.

The activity of a miRNA mimic can be assayed by detecting the suppression, at least partially, of the expression of a target gene, as manifested by a reduction of the amount of target mRNA, which may be isolated from a first cell or group of cells in which a target gene is transcribed and which has or have been treated such that the expression of a target gene is inhibited, as compared to a second cell or group of cells substantially identical to the first cell or group of cells but which has or have not been so treated (control cells). The degree of inhibition is usually expressed in terms of:

*[(mRNA in control cells) - (mRNA in treated cells) *100 percent] / (mRNA in control cells)*

When the miRNA mimic is used in vivo, the levels of target mRNA can be determined in a biological sample, e.g., a blood or serum, or urine or CSF sample either before and after administration of the miRNA mimic or in animals which have been treated using untreated animals as controls.

Alternatively, the degree of inhibition may be given in terms of a reduction of a parameter that is functionally linked to target gene expression, e.g., the amount of protein encoded by a target mRNA or the number of cells displaying a certain phenotype. In principle, target genome silencing may be determined in any cell expressing the target mRNA, either constitutively or by genomic engineering, and by any appropriate assay. However, when a reference is needed in order to determine whether a given miRNA mimic inhibits the expression of a target mRNA by a certain degree and therefore is encompassed by the instant invention, the assay provided in the Examples below and those known in the art shall serve as such reference. For example, in certain instances, expression of a target mRNA is suppressed by at least about 5 percent, 10 percent, 15 percent, 20 percent, 25 percent, 30 percent, 35 percent, 40 percent, 45 percent, or 50 percent by administration of the miRNA mimic. In some embodiments, a target mRNA is suppressed by at least about 60 percent, 70 percent, or 80 percent by administration of the miRNA mimic. In some embodiments, the target gene is suppressed by at least about 85 percent, 90 percent, or 95 percent by administration of the miRNA mimic.

The level of the target mRNA level can be detected by using RT-PCR, Northern blot or any other standard methods). Alternatively, the level of the polypeptide encoded by the target mRNA can be measured using Western blot, ELISA or any other immunological or non-immunological method. A substantial change in the expression level of mRNA or of the protein encoded by the target mRNA after the introduction of the miRNA mimic sequence is indicative of the effectiveness of the mimic sequence in suppressing the expression of the target gene. In one specific example, the expression levels of other genes are also monitored before and after the introduction of the miRNA mimic sequence. A miRNA mimic which has inhibitory effect on target gene expression but does not significantly affect the expression of other mRNAs can be selected.

Suitable target mRNAs that can be detected to measure the function of the miRNA mimics include:

- The c-Met mRNA in the case of the miR-34a mimic,
- The MITF mRNA, the BC12 mRNA and/or the cyclin D2 mRNA in the case of the miR-182 mimic,
- The MITF mRNA, the CDK2 mRNA and/or the CDK6 mRNA in the case of the miR-137 mimic and
- The c-Met mRNA in the case of the miR-144 mimic.

In a preferred embodiment, the mimics forming part of the composition according to the present invention mimic the human miRNAs. A human miRNA molecule is a miRNA molecule which is found in a human cell, tissue or organ. A human miRNA molecule may also be a human miRNA molecule derived from an endogenous human miRNA molecule by substitution, deletion and/or addition of a nucleotide. Thus, in a preferred embodiment, the miRNA-34a mimic is a hsa-miR-34a mimic. In another embodiment, the miRNA-182 mimic is a hsa-miR-182 mimic . In another embodiment, the miRNA-137 mimic is a hsa-miR-137 mimic. In another embodiment, the miRNA-144 mimic is a hsa-mir-144 mimic.

Moreover, each pre-miRNA can produce mature miRNAs from the 5' arm or from the 3' arm and thus, the resulting miRNAs are designated with the suffixes 5p or 3p, respectively. In the context of the present invention, the terms miRNA-34a, miR-182, miRNA-137 and miRNA-144 are to be understood as the 5p versions thereof, i.e. the mature miRNAs derive in each case from the 5' arm of the corresponding pre-miRNAs.

Thus, in a preferred embodiment, the miR-34 mimic is a hsa-miR-34-5p mimic. In a preferred embodiment, the miR-182 mimic is a hsa-miR-182-5p mimic. In a preferred embodiment, the miR-137 mimic is a hsa-miR-137-5p mimic. In a preferred embodiment, the miR-144 mimic is a hsa-miR-144-5p mimic.

The composition according to the invention comprises mimics of the miRNAs shown in Table 1:

Table 1: Accession numbers and sequences of the miRNAs which are mimicked by the miRNA mimics found in the compositions according to the present invention. The column labelled "Sequence" shows the sequence of the pre-miRNA, wherein the sequence of the mature miRNA is underlined.

| miRNA | Accession code pre-miRNA | Accession code mature miRNA | Sequence | SEQ ID NO: (pre-miRNA) | SEQ ID NO: (mature miRNA) |
|---|---|---|---|---|---|
| miRNA-34a | MI0000268 | MIMAT0000255 | 1 GGCCAGCUGU GAGUGUUUCU UUGGCAGUGU CUUAGCUGGU UGUUGUGAGC<br>51 AAUAGUAAGG AAGCAAUCAG CAAGUAUACU GCCCUAGAAG UGCUGCACGU<br>101 UGUGGGGCCC | 1 | 5 |
| miRNA-182 | MI0000272 | MIMAT0000259 | 1 GAGCUGCUUG CCUCCCCCCG UUUUUGGCAA UGGUAGAACU CACACUGGUG<br>51 AGGUAACAGG AUCCGGUGGU UCUAGACUUG CCAACUAUGG GGCGAGGACU<br>101 CAGCCGGCAC | 2 | 6 |
| miRNA-137 | MI0000454 | MIMAT0000429 | 1 GGUCCUCUGA CUCUCUUCGG UGACGGGUAU UCUUGGGUGG AUAAUACGGA<br>51 UUACGUUGUU AUUGCUUAAG AAUACGCGUA GUCGAGGAGA GUACCAGCGG<br>101 CA | 3 | 7 |
| miRNA-144 | MI0000460 | MIMAT0004600 | 1 UGGGGCCCUG GCUGGGAUAU CAUCAUAUAC UGUAAGUUUG CGAUGAGACA<br>51 CUACAGUAUA GAUGAUGUAC UAGUCCGGGC ACCCCC | 4 | 8 |

*DNA-based miRNA mimics*

In one embodiment, one or more of the miRNA mimics present in the composition according to the invention is a DNA oligonucleotide comprising a DNA version of the sequence of the mature miRNA or a functionally variant equivalent thereof.

The term DNA oligonucleotide, as used herein, refers to a short DNA nucleic acid molecules having a sequence that is substantially complementary to a target mRNA or a portion thereof and has the ability to specifically hybridize to the target polynucleotide.

The term "short" refers to a length that is 20, 21, 22, 23, 24, 25, 26, 27, 28, 29, 30, 31, 32, 33, 34, 35, 36, 37, 38, 39, 40, 45, 50, 60, 70, 80, 90, 100, or 150 nucleotides or fewer, including all integers or ranges derivable there between. In various embodiments, the DNA oligonucleotide is between about 10 to 25 nucleotides in length and comprises a 5' to 3' sequence that is at least 90 percent identical to the 5' to 3' sequence of a mature miRNA. In certain embodiments, the DNA oligonucleotide is 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 60, 70, 80, 90, 100, or 150 nucleotides in length. Moreover, the DNA oligonucleotide may have a sequence (from 5' to 3') that is or is at least 50, 60, 70, 75, 80, 85, 90, 91, 92, 93, 94, 95, 96, 97, 98, 99, 99.1, 99.2, 99.3, 99.4, 99.5, 99.6, 99.7, 99.8, 99.9 or 100 percent identical to the 5' to 3' sequence of a mature miRNA, particularly a mature, naturally occurring miRNA.

The DNA oligonucleotides according to the present invention comprise, consists essentially or consists of a DNA sequence which is the DNA version of the sequence of the mature miRNA which is mimicking or a of functionally equivalent variant thereof. The term "DNA version" of a given RNA sequence refers to a sequence which is the same as the RNA sequence wherein the U residues are replaced by T residues. Accordingly, for a RNA sequence:
UUGGCAGUGU CUUAGCUGGU UGU (SEQ ID NO:5) the DNA version thereof will be the sequence:
TTGGCAGTGT CTTAGCTGGT TGT (SEQ ID NO:9)

It will be understood that the DNA oligonucleotides need not show a complete identity with the sequence of the mature miRNA that is mimicking but that the sequence of the DNA oligonucleotide can be substantially identical to the sequence of the miRNA. By "substantially identical" is meant a nucleic acid molecule exhibiting at least 50 percent identity to a reference nucleic acid sequence (for example, any one of the nucleic acid sequences described herein). In certain embodiments, the sequence of the DNA oligonucleotide has at least 80%, at least 85%, at least 90%, at least 95% or more with the sequence of the miRNA. In certain embodiments, the nucleobase sequence of the DNA oligonucleotide has 100 % identity with the nucleobase sequence of the miRNA which is mimicking. In determining sequence identity between a DNA oligonucleotide and the mature miRNA, matching positions are considered those wherein the same nucleotide is present in the same position or when the position in the DNA is a T nucleobase, then the corresponding position in the miRNA is an U.

Sequence identity is typically measured using sequence analysis software (for example, Sequence Analysis Software Package of the Genetics Computer Group, University of Wisconsin Biotechnology Center, 1710 University Avenue, Madison, Wis. 53705, BLAST, BESTFIT, GAP, or PILEUP/PRETTYBOX programs). Such software matches identical or similar sequences by assigning degrees of homology to various substitutions, deletions, and/or other modifications. Conservative amino acid substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine. In an exemplary approach to determining the degree of identity, a BLAST program may be used, with a probability score between E=3 and E=100 indicating a closely related sequence.

In a preferred embodiment, the DNA oligonucleotide mimic is capable of hybridizing with at the mRNA which is targeted by the miRNA that is being mimicked by the DNA. By "hybridize" is meant pair to form a double-stranded molecule between complementary polynucleotide sequences (e.g., a gene described herein), or portions thereof, under various conditions of stringency. In one embodiment, therapeutic oligonucleotides hybridize in physiological buffer at 37°C in patients.

For example, stringent salt concentration will ordinarily be less than about 750 mM NaCl and 75 mM trisodium citrate, preferably less than about 500 mM NaCl and 50 mM trisodium citrate, and more preferably less than about 250 mM NaCl and 25 mM trisodium citrate. Low stringency hybridization can be obtained in the absence of organic solvent, e.g., formamide, while high stringency hybridization can be obtained in the presence of at least about 35 percent formamide, and more preferably at least about 50 percent formamide. Stringent temperature conditions will ordinarily include temperatures of at least about 30 degrees centigrade, more preferably of at least about 37 degrees centigrade, and most preferably of at least about 42 degrees centigrade Varying additional parameters, such as hybridization time, the concentration of detergent, e.g., sodium dodecyl sulfate (SDS), and the inclusion or exclusion of carrier DNA, are well known to those skilled in the art. Various levels of stringency are accomplished by combining these various conditions as needed. In a preferred: embodiment, hybridization will occur at 30 degrees centigrade in 750 mM NaCl, 75 mM trisodium citrate, and 1 percent SDS. In a more preferred embodiment, hybridization will occur at 37 degrees centigrade in 500 mM NaCl, 50 mM trisodium citrate, 1 percent SDS, 35 percent formamide, and 100 micro g/ml denatured salmon

sperm DNA (ssDNA). In a most preferred embodiment, hybridization will occur at 42 degrees centigrade in 250 mM NaCl, 25 mM trisodium citrate, 1 percent SDS, 50 percent formamide, and 200 micro g/ml ssDNA. Useful variations on these conditions will be readily apparent to those skilled in the art.

For most applications, washing steps that follow hybridization will also vary in stringency. Wash stringency conditions can be defined by salt concentration and by temperature. As above, wash stringency can be increased by decreasing salt concentration or by increasing temperature. For example, stringent salt concentration for the wash steps will preferably be less than about 30 mM NaCl and 3 mM trisodium citrate, and most preferably less than about 15 mM NaCl and 1.5 mM trisodium citrate. Stringent temperature conditions for the wash steps will ordinarily include a temperature of at least about 25°C, more preferably of at least about 42°C, and even more preferably of at least about 68°C. In a preferred embodiment, wash steps will occur at 25 degrees centigrade in 30 mM NaCl, 3 mM trisodium citrate, and 0.1 percent SDS. In a more preferred embodiment, wash steps will occur at 42°C in 15 mM NaCl, 1.5 mM trisodium citrate, and 0.1 percent SDS. In a more preferred embodiment, wash steps will occur at 68 degrees centigrade in 15 mM NaCl, 1.5 mM trisodium citrate, and 0.1 percent SDS. Additional variations on these conditions will be readily apparent to those skilled in the art. Hybridization techniques are well known to those skilled in the art and are described, for example, in Benton and Davis (Science 196:180, 1977); Grunstein and Hogness (Proc. Natl. Acad. Sci., USA 72:3961, 1975); Ausubel et al. (Current Protocols in Molecular Biology, Wiley Interscience, New York, 2001); Berger and Kimmel (Guide to Molecular Cloning Techniques, 1987, Academic Press, New York); and Sambrook et al., Molecular Cloning: A Laboratory Manual, Cold Spring Harbor Laboratory Press, New York.

In a preferred embodiment, the DNA-based miRNA mimic comprises the DNA version of the seed sequence of miRNA-34a, of miRNA-182, of miRNA-137 and of miRNA-144.

In a more preferred embodiment, the miRNA-34a mimic is a DNA molecule the sequence of which comprises, essentially consists or consists of the sequence of the mature miRNA-34a as defined in the miRbase database under accession number MIMAT0000255 or a functionally variant equivalent thereof, the miRNA-182 mimic is a DNA molecule the sequence of which comprises, essentially consists or consists of the sequence of the mature miRNA-182 as defined in the miRbase database under accession number MIMAT0000259, the miRNA-137 mimic is a DNA molecule the sequence of which comprises, essentially consists or consists of the sequence mature miRNA-137 as defined in the miRbase database under accession number MIMAT0000429 and/or the miRNA-144 mimic is a DNA molecule the sequence of which comprises, essentially consists or consists of the sequence of mature miRNA-144 as defined in the miRbase database under accession number MIMAT0004600.

In preferred embodiments, the combination according to the invention comprises DNA-based mimics wherein the miRNA-34a mimic comprises a sequence shown in SEQ ID NO: 5, the miRNA-182 mimic comprises a sequence shown in SEQ ID NO: 6, the miRNA-137 mimic comprises a sequence shown in SEQ ID NO: 7 and the miRNA-144 mimic comprises a sequence shown in SEQ ID NO: 8.

In preferred embodiments, the combination according to the invention comprises DNA-based mimics wherein the miRNA-34a mimic comprises a sequence shown in SEQ ID NO: 9, the miRNA-182 mimic comprises a sequence shown in SEQ ID NO: 10, the miRNA-137 mimic comprises a sequence shown in SEQ ID NO: 1 and the miRNA-144 mimic comprises a sequence shown in SEQ ID NO: 12 which correspond respectively to the sequences of SEQ ID NO:5, 6, 7 and 8 but modified in their 3' terminus by the addition of a 4 polyadenine stretch.

The DNA-based miRNA mimics according to the present invention may contain modifications that make them more resistant to nuclease activities. These modifications include modifications of the phosphate backbone and modifications of sugars within the nucleobases.

Examples of modifications contemplated for the internucleoside linkages in the phosphate backbone include boranophosphate, methylphosphonate, phosphorothioate, phosphoramidate, phosphorodiamidate, phosphorodithioate, phosphoroselenoate, phosphorodiselenoate, phosphoroanilothioate and phosphoranilidate, or any combinations thereof. Boranophosphate modified RNAs are highly nuclease resistant, potent as silencing agents, and are relatively non-toxic. Phosphorothioate and methylphosphonate modifications can be readily placed in a nucleic acid molecule of the invention at any desired position and can be made using standard chemical synthesis methods.

Modifications to one or more backbone residues of the nucleic acids may comprise one or more of the following: 2' sugar modifications include 2'-O-alkyl such as 2'-O-methyl (2'-OMe), 2'-O-methoxyethyl (2'-MOE), 2'-O-methoxyethoxy, 2'- Fluoro (2'-F), 2'-Allyl, 2'-O-[2-(methylamino)-2-oxoethyl], 2'-O-(N-methylcarbamate); 4' sugar modifications including 4'-thio, 4'-$CH_2$-O-2'-bridge, 4-$(CH_2)_2$-O-2'-bridge; Locked Nucleic Acid (LNA); Peptide Nucleic Acid (PNA); Intercalating nucleic acid (INA); Twisted intercalating nucleic acid (TINA); Hexitol nucleic acids (HNA); arabinonucleic acid (ANA); cyclohexane nucleic acids (CNA); cyclohexenylnucleic acid (CeNA); threosyl nucleic acid (TNA); Morpholino oligonucleotides; Gap-mers; Mix-mers; Incorporation Arginine-rich peptides; addition of 5'-phosphate to synthetic RNAs; RNA Aptamers (Que-Gewirth NS, Gene Ther. 2007 Feb;14(4):283-91.); RNA Aptamers regulated with antidotes on the subject of the specific RNA aptamer (ref. Oney S, Oligonucleotides. 2007 Fall;17(3):265-74.) or any combinations thereof. A variety of substitutions can be placed at the 2'-position of the ribose. Such 2' modifications generally increase duplex stability (Tm) and can greatly improve nuclease resistance. Examples of modifications contemplated for the sugar moiety

include 2'-O-alkyl, such as 2'-O-methyl, 2'-fluoro, 2'-amino modifications and the like. Examples of modifications contemplated for the base groups include abasic sugars, modified pyrimidines, modified purines, and the like.

In another embodiment, the DNA-based miRNA mimics are locked nucleic acids (LNAs), which are a particular class of 2'-modification that can be incorporated to stabilize nucleic acid molecules of the invention. Many other modifications are known and can be used so long as the above criteria are satisfied.

In another embodiment, the DNA-based miRNA mimics are peptide nucleic acids (PNAs) which are chemically synthesized oligoamides of N-aminoethyl glycine with nucleic acid bases attached to the alpha amine of glycine.

*RNA-based miRNA mimics*

In another embodiment, one or more of the miRNA mimics present in the composition according to the invention is/are RNA-based mimics, which are double-stranded RNAs, wherein the sequence of one of the strands comprises the sequence of the mature miRNA or a functionally variant equivalent thereof. The strand containing the sequence which comprises the sequence of the miRNA is known as the "guide strand", whereas the complementary strand known as the "passenger strand".

The term "double-stranded RNA", as used herein, refers to a RNA molecule which contains at least two regions which have sequences that are at least partially complementary to each other so that they can form a double-stranded region. The two regions containing the at least partially complementary sequences may be forming part of a single RNA molecule if they are connected by a linker sequence, in which case the dsRNA will have a stem-loop structure of the two regions may be present in different RNA molecules, thereby resulting in a double-stranded RNA formed by intermolecular base pairing. In a preferred embodiment, the dsRNA is formed by two different RNA molecules. Double-stranded RNAs also include partially double-stranded RNAs, which are double-stranded structures that also comprise single-stranded structures at the 5' and/or at the 3' end, which occurs when each strand of a miRNA molecule does not have the same length, thereby resulting in the presence of overhangs. In general, such partial double-stranded miRNA molecule may have less than 75 percent double-stranded structure and more than 25 percent single stranded structure, or less than 50 percent double-stranded structure and more than 50 percent single stranded structure, or more preferably less than 25 percent, 20 percent or 15 percent double-stranded structure and more than 75 percent, 80 percent, 85 percent single-stranded structure.

In this case, the dsRNA formed by base pairing of the two RNA molecules may be blunt ended or may contain overhangs. The overhangs can consist of 1-6 nucleotides on either the 3' or 5' end of either strand and can be modified to enhance stability or functionality. The overhangs in the dsRNA may be a 5' overhang of the passenger strand, a 3' overhang in the passenger strand, a 5' overhang in the guide strand and/or a 3' overhang in the guide strand. In one embodiment, a miRNA mimic comprises a duplex region of between 16 and 31 nucleotides and one or more of the following chemical modification patterns: the sense strand contains 2'-O-methyl modifications of nucleotides 1 and 2 (counting from the 5' end of the sense oligonucleotide), and all of the Cs and Us; the antisense strand modifications can comprise 2' F modification of all of the Cs and Us, phosphorylation of the 5' end of the oligonucleotide, and stabilized internucleotide linkages associated with a 2 nucleotide 3' overhang.

It will be understood that the sequence of the guide strand need not be completely identical to the sequence of the mature miRNA. If not completely identical, the guide strand in the dsRNA miRNA mimics is at least substantially identical to the sequence of the mature miRNA.

In one embodiment, the miR-34a mimic is a RNA molecule containing the sequence of the mature miR-34a. In another embodiment, the miR-34a mimic is a RNA molecule which comprises the seed sequence of miR-34a.

In one embodiment, the miRNA-182 mimic is a RNA molecule containing the sequence of the mature miRNA-182. In another embodiment, the miR-182 mimic is a RNA molecule which comprises the seed sequence of miR-182.

In one embodiment, the miRNA-137 mimic is a RNA molecule containing the sequence of the mature miRNA-137. In another embodiment, the miR-137 mimic is a RNA molecule which comprises the seed sequence of miR-137.

In one embodiment, the miRNA-144 mimic is a RNA molecule containing the sequence of the mature miRNA-144. In another embodiment, the miR-144 mimic is a RNA molecule which comprises the seed sequence of miR-144.

In some embodiments, the miRNA mimic is an RNA molecule that is double-stranded, meaning the molecule is composed of two polynucleotides or strands that can be separated from one another. A double-stranded molecule does not include a hairpin molecule, which is one strand or polynucleotide. In some embodiments, the RNA molecule is blunt-ended on one or both ends. In a double-stranded RNA molecule, one or both strands may be 18, 19, 20, 21, 22, 23, 24, or 25 nucleotides in length, or any range derivable therein. In certain embodiments, a double-stranded, blunt-ended molecule is 22 or 23 base pairs (bps) in length.

It is contemplated that in some embodiments a double-stranded RNA molecule contains two strands that are fully complementary to one another, which results in a molecule that is necessarily blunt-ended.

In a preferred embodiment, the miRNA mimic comprises at least 6 of the 7 nucleotides present in the seed sequence of the miRNA molecule or equivalent Preferably in this embodiment, a miRNA mimic can be from 6, 7, 8, 9,

10, 11, 12 to 30 nucleotides in length and more preferably comprises at least 6 of the 7 nucleotides present in the seed sequence of said miRNA molecule or equivalent thereof. Even more preferably a miRNA mimic is from 15 to 28 nucleotides in length and more preferably comprises at least 6 of the 7 nucleotides present in the seed sequence. Even more preferably a miRNA mimic has a length of at least 6, 7, 8, 9, 10, 11, 12, 13, 14, 15, 16, 17, 18, 19, 20, 21, 22, 23, 24, 25, 26, 27, 28, 29, 30 nucleotides or more and preferably comprises at least 6 of the 7 nucleotides present in the seed sequence.

In one embodiments, the guide strand in the RNA-based miRNA mimic may comprise 7 nucleotides of the sequence of the mature miRNA. Even more preferably the guide strand in the RNA-based miRNA mimic has at least 7, 8, 9, 10, 11, 12, 13, 14, 15, 16, 17, 18, 19, 20, 21, 22, 23, 24, 25, 26, 27, 28, 29, 30 nucleotides or more and comprises the 7 nucleotides present in the seed sequence as identified in table 1.

In another preferred embodiment, the RNA-based miRNA mimic comprises at least 6 of the 7 nucleotides present in a given seed sequence as identified in table 1 and has at least 80 percent identity over the whole mature sequence. Preferably, identity is at least 80 percent, 81 percent, 82 percent, 83 percent, 84 percent, 85 percent, 86 percent, 87 percent, 88 percent, 89 percent, 90 percent, 91 percent, 92 percent, 93 percent, 94 percent, 95 percent, or higher such as 96 percent, 97 percent, 98 percent, 99 percent or 100 percent.

Alternatively, preferably in this embodiment, the RNA-based miRNA mimic has a length of not more than 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 nucleotides, comprises at least 6 of the 7 nucleotides present in a given seed sequence as identified in table 1 and has at least 80 percent identity over the whole mature sequence as identified in table 3 as SEQ ID NO: 2-21. Preferably, identity is at least 80 percent, 81 percent, 82 percent, 83 percent, 84 percent, 85 percent, 86 percent, 87 percent, 88 percent, 89 percent, 90 percent, 91 percent, 92 percent, 93 percent, 94 percent, 95 percent, 96 percent, 97 percent, 98 percent, 99 percent or 100 percent.

Sequence identity may be assessed using several ways as defined above. In one embodiment, the combination according to the invention is formed by double-stranded inhibitory RNA-based miRNA mimics wherein the mimics comprise two anticomplementary RNA sequence regions and wherein the:

- The miRNA34a mimic comprises a RNA sequence region corresponding to the mature miRNA-34a as defined in the miRbase database under accession number MIMAT0000255 or a functionally variant equivalent thereof,
- the miRNA-182 mimic comprises a RNA sequence region corresponding to the mature miRNA-182 as defined in the miRbase database under accession number MIMAT0000259 or a functionally variant equivalent thereof,
- the miRNA-137 mimic comprises a RNA sequence region corresponding to the mature miRNA-137 as defined in the miRbase database under accession number MIMAT0000429 or a functionally variant equivalent thereof and/or
- the miRNA-144 mimic comprises a RNA sequence region corresponding to the mature miRNA-144 as defined in the miRbase database under accession number MIMAT0004600 or a functionally variant equivalent thereof.

In yet another embodiment, the miRNA34a mimic comprises a first sequence region comprising the sequence shown in SEQ ID NO: 17 or a functionally variant equivalent thereof and a second sequence region comprising the sequence shown in SEQ ID NO: 18 or a functionally variant equivalent thereof, the miRNA-182 mimic comprises a first sequence region comprising the sequence shown in SEQ ID NO: 19 or a functionally variant equivalent thereof and a second sequence region comprising the sequence shown in SEQ ID NO: 20 or a functionally variant equivalent thereof, the miRNA-137 mimic comprises a first sequence region comprising the sequence shown in SEQ ID NO: 21 or a functionally variant equivalent thereof and a second sequence region comprising the sequence shown in SEQ ID NO: 22 or a functionally variant equivalent thereof and wherein the miRNA-144 mimic comprises a first sequence region comprising the sequence shown in SEQ ID NO: 23 or a functionally variant equivalent thereof and a second sequence region comprising the sequence shown in SEQ ID NO: 24 or a functionally variant equivalent thereof.

The invention encompasses stabilized double-stranded inhibitory RNAs having modifications that protect against 3' and 5' exonucleases as well as endonucleases. Such modifications desirably maintain target affinity while increasing stability in vivo. In various embodiments, the double-stranded inhibitory RNAs of the invention include chemical substitutions at the ribose and/or phosphate and/or base positions of a given nucleobase sequence. For example, the double-stranded inhibitory RNAs of the invention include chemical modifications at the 2' position of the ribose moiety, circularization of the aptamer, 3' capping and 'spiegelmer' technology. The double-stranded inhibitory RNAs having A and G nucleotides sequentially replaced with their 2'-$OCH_3$ modified counterparts are particularly useful in the methods of the invention. Such modifications are typically well tolerated in terms of retaining affinity and specificity. In various embodiments, the double-stranded inhibitory RNAs include at least 10 percent, 25 percent, 50 percent, or 75 percent modified nucleotides. In other embodiments, as many as 80-90 percent of the double-stranded inhibitory RNAs nucleotides contain stabilizing substitutions. In other embodiments, 2'-OMe containing double-stranded inhibitory RNAs are synthesized. Such double-stranded inhibitory RNAs are desirable because they are inexpensive to synthesize and natural polymerases do not accept 2'-OMe nucleotide triphosphates as substrates so that 2'-OMe nucleotides cannot

be recycled into host DNA. Using methods described herein, double-stranded inhibitory RNAs will be selected for increased in vivo stability. In one embodiment, double-stranded inhibitory RNAs having 2'-F and 2'-$OCH_3$ modifications are used to generate nuclease resistant aptamers. In other embodiments, the nucleic acids of the invention have one or more locked nucleic acids (LNA). LNA refers to a modified RNA nucleotide. The ribose of the LNA is modified with an extra bridge connecting the 2' oxygen and the 4' carbon which locks the ribose into the North or 3'-endo conformation. In other embodiments, one or more nucleic acids of the invention incorporate a morpholino structure where the nucleic acid bases are bound to morpholine rings instead of deoxyribose rings and are linked through phosphorodiamidate groups instead of phosphates. Yet other modifications, include (PS)-phosphate sulfur modifications wherein the phosphate backbone of the nucleic acid is modified by the substitution of one or more sulfur groups for oxygen groups in the phosphate backbone.

*Polynucleotides encoding a miRNA or a precursor thereof*

In another embodiment, one or more of the miRNA mimics present in the composition according to the invention is a polynucleotide encoding the miRNA or a precursor thereof.

In one embodiment, the polynucleotide encodes a miRNA precursor molecule, which can be the pri-miRNA or the pre-miRNA. The miRNA precursor can also be a precursor RNA that encodes a pri-miRNA comprising said miRNA stem-loop sequence flanked on each side by at least 30 single-stranded RNA nucleotides at the 5' and 3' sides of the miRNA stem-loop sequence.

The terms "polynucleotide," "polynucleotide sequence," "nucleic acid sequence," and "nucleic acid fragment" are used interchangeably herein. These terms encompass nucleotide sequences and the like. A polynucleotide may be a polymer of RNA or DNA that is single- or double-stranded, that optionally contains synthetic, non-natural or altered nucleotide bases. A polynucleotide in the form of a polymer of DNA may be comprised of one or more segments of cDNA, genomic DNA, synthetic DNA, or mixtures thereof. The use of the term "polynucleotide" is not intended to limit the present invention to polynucleotides comprising DNA. Those of ordinary skill in the art will recognize that polynucleotides, can comprise ribonucleotides and combinations of ribonucleotides and deoxyribonucleotides. Such deoxyribonucleotides and ribonucleotides include both naturally occurring molecules and synthetic analogues. The polynucleotides of the invention also encompass all forms of sequences including, but not limited to, single-stranded forms, double-stranded forms, hairpins, stem-and-loop structures, and the like.

The compositions provided herein can comprise an isolated or substantially purified polynucleotides encoding the miRNAs. An "isolated" or "purified" polynucleotide is substantially or essentially free from components that normally accompany or interact with the polynucleotide as found in its naturally occurring environment. Thus, an isolated or purified polynucleotide is substantially free of other cellular material, or culture medium when produced by recombinant techniques, or substantially free of chemical precursors or other chemicals when chemically synthesized. Optimally, an "isolated" polynucleotide is free of sequences (optimally protein encoding sequences) that naturally flank the polynucleotide (i.e., sequences located at the 5' and 3' ends of the polynucleotide) in the genomic DNA of the organism from which the polynucleotide is derived. For example, in various embodiments, the isolated polynucleotide can contain less than about 5 kb, 4 kb, 3 kb, 2 kb, 1 kb, 0.5 kb, or 0.1 kb of nucleotide sequence that naturally flank the polynucleotide in genomic DNA of the cell from which the polynucleotide is derived.

In some embodiments, the polynucleotide encoding the precursor of the miRNA and which forms part of the composition of the present invention encodes a precursor of a miRNA molecule that has at least 80 percent identity with said sequence as identified in table 1. Preferably, the identity is at least 80 percent, 81 percent, 82 percent, 83 percent, 84 percent, 85 percent, 86 percent, 87 percent, 88 percent, 89 percent 90 percent, 91 percent, 92 percent, 93 percent, 94 percent, 95 percent, 96 percent, 97 percent, 98 percent, 99 percent or 100 percent. Preferably in this embodiment, the polynucleotide has a length of at least 50, 55, 60, 70, 75, 80, 85, 90, 95, 100, 130, 150, 200, 250, 300, 350, 400 nucleotides or more and has at least 80 percent, 81 percent, 82 percent, 83 percent, 84 percent, 85 percent, 86 percent, 87 percent, 88 percent, 89 percent 90 percent, 91 percent, 92 percent, 93 percent, 94 percent, 95 percent, 96 percent, 97 percent, 98 percent, 99 percent or 100 percent identity with a DNA sequence encoding the precursor of the miRNA molecules identified in table 1 as SEQ ID NO: 1 to 4.

Further provided are recombinant polynucleotides comprising the miRNA expression constructs and various components thereof. The terms "recombinant polynucleotide" and "recombinant DNA construct" are used interchangeably herein. A recombinant construct comprises an artificial or heterologous combination of nucleic acid sequences, e.g., regulatory and coding sequences that are not found together in nature. In one embodiment, a recombinant construct may comprise regulatory sequences and coding sequences that are derived from different sources, or regulatory sequences and coding sequences derived from the same source, but arranged in a manner different than that found in nature. Such a construct may be used by itself or may be used in conjunction with a vector. If a vector is used, then the choice of vector is dependent upon the method that will be used to transform host cells as is well known to those skilled in the art. For example, a plasmid vector can be used. The skilled artisan is well aware of the genetic elements that must

be present on the vector in order to successfully transform, select and propagate host cells comprising any of the isolated nucleic acid fragments of the invention.

In specific embodiments, one or more of the miRNA expression constructs described herein can be provided in an expression cassette for expression in the organism or cell type of interest. The cassette can include 5' and 3' regulatory sequences operably linked to a polynucleotide provided herein. "Operably linked" is intended to mean a functional linkage between two or more elements. For example, an operable linkage between a polynucleotide of interest and a regulatory sequence (i.e., a promoter) is a functional link that allows for expression of the polynucleotide of interest. Operably linked elements may be contiguous or non-contiguous. When used to refer to the joining of two protein coding regions, by operably linked is intended that the coding regions are in the same reading frame. The cassette may additionally contain at least one additional gene to be co-transformed into the organism. Alternatively, the additional gene(s) can be provided on multiple expression cassettes. Such an expression cassette is provided with a plurality of restriction sites and/or recombination sites for insertion of a recombinant polynucleotide to be under the transcriptional regulation of the regulatory regions. The expression cassette may additionally contain selectable marker genes.

The expression cassette can include in the 5'-3' direction of transcription, a transcriptional and translational initiation region (i.e., a promoter), a recombinant polynucleotide provided herein, and a transcriptional and translational termination region (i.e., termination region) functional in the desired host. The regulatory regions (i.e., promoters, transcriptional regulatory regions, and translational termination regions) and/or a recombinant polynucleotide provided herein may be native/analogous to the host cell or to each other. Alternatively, the regulatory regions and/or a recombinant polynucleotide provided herein may be heterologous to the host cell or to each other. For example, a promoter operably linked to a heterologous polynucleotide is from a species different from the species from which the polynucleotide was derived, or, if from the same/analogous species, one or both are substantially modified from their original form and/or genomic locus, or the promoter is not the native promoter for the operably linked polynucleotide. Alternatively, the regulatory regions and/or a recombinant polynucleotide provided herein may be entirely synthetic.

The termination region may be native with the transcriptional initiation region, may be native with the operably linked recombinant polynucleotide of interest, may be native with the host, or may be derived from another source (i.e., foreign or heterologous) to the promoter, the recombinant polynucleotide of interest, the host, or any combination thereof.

A number of promoters can be used in the miRNA expression constructs provided herein. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. The promoters can be selected based on the desired outcome. It is recognized that different applications can be enhanced by the use of different promoters in the miRNA expression constructs to modulate the timing, location and/or level of expression of the miRNA. Such miRNA expression constructs may also contain, if desired, a promoter regulatory region (e.g., one conferring inducible, constitutive, environmentally- or developmentally-regulated, or cell- or tissue-specific/selective expression), a transcription initiation start site, a ribosome binding site, an RNA processing signal, a transcription termination site, and/or a polyadenylation signal.

In some embodiments, a miRNA expression construct provided herein can be combined with constitutive, tissue-preferred, or other promoters for expression in the cell of interest.

The polynucleotide encoding the miRNA according to the invention can be operatively coupled to a promoter allowing the transcription of said polynucleotide in the cell in which the miRNA is to be expressed. Promoters suitable for carrying out the present invention include, without necessarily being limited to, constitutive promoters such as the U1 promoter or promoters derived from eukaryotic virus genomes such as the polyomavirus, adenovirus, SV40, CMV, the avian sarcoma virus, the hepatitis B virus, the metallothionein gene promoter, the thymidine kinase gene promoter of the herpes simplex virus, LTR regions of retroviruses, the immunoglobulin gene promoter, the actin gene promoter, the EF-1 alpha gene promoter, the mouse mammary tumor virus (MMTV), the human immunodeficiency virus (HIV) long terminal repeat (LTR) promoter, the MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter as well as inducible promoters in which the expression of the gene depends on the addition of a molecule or of an exogenous signal, such as the tetracycline system, the NF-kappa B/UV light system, the Cre/Lox system and the heat-shock gene promoter, the regulatable RNA polymerase II promoters described in WO/2006/135436, as well as tissue-specific promoters (for example, the PSA promoter described in WO2006012221). In a preferred embodiment, the promoters are the RNA polymerase II promoters acting in a constitutive manner.

It is also possible to use tissue-specific promoters in the event that the gene the expression of which is to be silenced is specifically expressed in a tissue:

- a specific stomach promoter, such as the H/K-ATPase beta-subunit promoter, the K19 promoter, the metallothionein promoter, the TFF1 promoter, the TFF2 promoter, the FOXa/HNF2 gamma promoter,

- a specific pancreas promoter, such as the elastase promoter, the Pdx-1 promoter, the insulin promoter or the phosphoglycerate kinase promoter,
- a specific lung promoter such as the Clara cell secretory protein promoter, the surfactant protein C promoter,
- a specific breast promoter, such as the mouse mammary tumour virus promoter or the whey acidic protein promoter,
- a specific skin promoter, including the keratin promoter or the K14 promoter,
- a specific oesophagus promoter, such as EBV promoter 12,
- a specific liver promoter, such as the major urinary protein promoter or the albumin promoter,
- a specific colon promoter, such as the villin promoter or the FABP-TS4 promoter,
- a specific prostrate promoter, such as the cryptidin-2 promoter, the prostate-specific antigen promoter, the C(3)1 promoter, the promoter of the protein secreted by the prostate with 94 amino acids (PSP94) or the probasin promoter,
- a specific kidney promoter, such as the uromodulin promoter, the Tamm-Horsfall protein promoter or the type 1 gamma-glutamyl transpeptidase promoter,
- a specific bladder promoter, such as the uroplakin promoter or the urohingin promoter,
- a specific uterus promoter, such as the uteroglobin promoter.

Additionally, any tissue-specific promoter described in the TiProD tissue-specific promoter database as described by Chen, X et al., (Nucleic Acids Res., 2006, 34, Database Issue) can be used in the context of the present invention.

An example of a promoter is the immediate early cytomegalovirus (CMV) promoter sequence. This promoter sequence is a strong constitutive promoter sequence capable of driving high levels of expression of any polynucleotide sequence operatively linked thereto. However, other. Further, the invention should not be limited to the use of constitutive promoters. Inducible promoters are also contemplated as part of the invention. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter.

The expression cassette containing the miRNA expression construct can also comprise a selectable marker gene for the selection of transformed cells. Selectable marker genes are utilized for the selection of transformed cells or tissues. Marker genes include genes encoding antibiotic resistance. Additional selectable markers include phenotypic markers such as beta-galactosidase and fluorescent proteins such as green fluorescent protein (GFP), cyan fluorescent protein (CYP) and yellow fluorescent protein

Vectors suitable for the insertion of said polynucleotides are vectors derived from expression vectors in prokaryotes such as pUC18, pUC19, Bluescript and the derivatives thereof, mp18, mp19, pBR322, pMB9, Co1E1, pCR1, RP4, phages and "shuttle" vectors such as pSA3 and pAT28, expression vectors in yeasts such as vectors of the type of 2 micron plasmids, integration plasmids, YEP vectors, centromere plasmids and the like, expression vectors in insect cells such as vectors of the pAC series and of the pVL, expression vectors in plants such as pIBI, pEarleyGate, pAVA, pCAMBIA, pGSA, pGWB, pMDC, pMY, pORE series and the like, and expression vectors in eukaryotic cells based on viral vectors (adenoviruses, viruses associated to adenoviruses such as retroviruses and, particularly, lentiviruses) as well as non-viral vectors such as pSilencer 4.1-CMV (Ambion), pcDNA3, pcDNA3.1/hyg, pHMCV/Zeo, pCR3.1, pEFI/His, pIND/GS, pRc/HCMV2, pSV40/Zeo2, pTRACER-HCMV, pUB6/V5-His, pVAX1, pZeoSV2, pCI, pSVL and PKSV-10, pBPV-1, pML2d and pTDT1.

The vectors preferably comprise a reporter or marker gene which allows identifying those cells that have been incorporated the vector after having been put in contact with it. Useful reporter genes in the context of the present invention include lacZ, luciferase, thymidine kinase, GFP and on the like. Useful marker genes in the context of this invention include, for example, the neomycin resistance gene, conferring resistance to the aminoglycoside G418; the hygromycin phosphotransferase gene, conferring resistance to hygromycin; the ODC gene, conferring resistance to the inhibitor of the ornithine decarboxylase (2-(difluoromethyl)-DL-ornithine (DFMO); the dihydrofolate reductase gene, conferring resistance to methotrexate; the puromycin-N-acetyl transferase gene, conferring resistance to puromycin; the ble gene, conferring resistance to zeocin; the adenosine deaminase gene, conferring resistance to 9-beta-D-xylofuranose adenine; the cytosine deaminase gene, allowing the cells to grow in the presence of N-(phosphonacetyl)-L-aspartate; thymidine kinase, allowing the cells to grow in the presence of aminopterin; the xanthine-guanine phosphoribosyltransferase gene, allowing the cells to grow in the presence of xanthine and the absence of guanine; the trpB gene of E. coli, allowing the cells to grow in the presence of indol instead of tryptophan; the hisD gene of E. coli, allowing the cells to use histidinol instead of histidine. The selection gene is incorporated into a plasmid that can additionally include a promoter suitable for the expression of said gene in eukaryotic cells (for example, the CMV or SV40 promoters), an optimized translation initiation site (for example, a site following the so-called Kozak's rules or an IRES), a polyadenylation site such as, for example, the SV40 polyadenylation or phosphoglycerate kinase site, introns such as, for example, the beta-globulin gene intron. Alternatively, it is possible to use a combination of both the reporter gene and the marker

gene simultaneously in the same vector.

In a preferred embodiment, the vectors are viral vectors. In an even more preferred embodiment, the vector used for the expression of the first or the second component of the invention (provided that it is based on DNA) is a viral vector that is selected from the group of adenoviruses, adeno-associated viruses, retroviruses, lentiviruses, herpes viruses, SV40 and alphaviruses.

Modifications of the miRNA mimics

In certain embodiments, the miRNA mimic forming part of the composition according to the invention is conjugated to one or more moieties which enhance the activity, cellular distribution or cellular uptake of the resulting antisense oligonucleotides. When the miRNA mimic is DNA-based or RNA-based comprising a single RNA polynucleotide stabilized by intrachain base pairing, the moiety may be connected to the 5' end or to the 3' end. When the miRNA mimic is RNA-based and double-stranded, the moiety may be connected to the 5' end of the passenger strand, to the 3' end of the guide strand, to the 5' end of the guide strand and/or to the 3' end of the guide strand.

In certain such embodiments, the moiety is a cholesterol moiety or a lipid moiety. Additional moieties for conjugation include carbohydrates, phospholipids, biotin, phenazine, folate, phenanthridine, anthraquinone, acridine, fluoresceins, rhodamines, coumarins, and dyes. In certain embodiments, a conjugate group is attached directly to an oligonucleotide. In certain embodiments, a conjugate group is attached to an oligonucleotide by a linking moiety selected from amino, hydroxyl, carboxylic acid, thiol, unsaturations (e.g., double or triple bonds), 8-amino-3,6-dioxaoctanoic acid (ADO), succinimidyl 4-(N-maleimidomethyl) cyclohexane-1-carboxylate (SMCC), 6-aminohexanoic acid (AHEX or AHA), substituted CI -CIO alkyl, substituted or unsubstituted C2-C10 alkenyl, and substituted or unsubstituted C2-C10 alkynyl. In certain such embodiments, a substituent group is selected from hydroxyl, amino, alkoxy, carboxy, benzyl, phenyl, nitro, thiol, thioalkoxy, halogen, alkyl, aryl, alkenyl and alkynyl.

In certain embodiments, the miRNA mimic is modified with a cap structure. This terminal modification protect an oligonucleotide from exonuclease degradation, and can help in delivery and/or localization within a cell. The cap can be present at the 5'-terminus (5'-cap), or at the 3'-terminus (3 '-cap), or can be present on both termini. Cap structures include, for example, inverted deoxy abasic caps. Suitable cap structures include a 4 ',5 '-methylene nucleotide, a 1-(beta-D-erythrofuranosyl) nucleotide, a 4'-thio nucleotide, a carbocyclic nucleotide, a 1,5-anhydrohexitol nucleotide, an L-nucleotide, an alpha-nucleotide, a modified base nucleotide, a phosphorodithioate linkage, a threopentofuranosyl nucleotide, an acyclic 3',4'-seco nucleotide, an acyclic 3,4-dihydroxybutyl nucleotide, an acyclic 3,5-dihydroxypentyl nucleotide, a 3 '-3 '-inverted nucleotide moiety, a 3 '-3'- inverted abasic moiety, a 3'-T-inverted nucleotide moiety, a 3'-T-inverted abasic moiety, a 1,4- butanediol phosphate, a 3'-phosphoramidate, a hexylphosphate, an aminohexyl phosphate, a 3'- phosphate, a 3'-phosphorothioate, a phosphorodithioate, a bridging methylphosphonate moiety, and a non-bridging methylphosphonate moiety 5'-amino-alkyl phosphate, a 1,3-diamino-2- propyl phosphate, 3-aminopropyl phosphate, a 6-aminohexyl phosphate, a 1,2-aminododecyl phosphate, a hydroxypropyl phosphate, a 5 '-5 '-inverted nucleotide moiety, a 5'-5'-inverted abasic moiety, a 5'-phosphoramidate, a 5'-phosphorothioate, a 5 '-amino, a bridging and/or non-bridging 5'-phosphoramidate, a phosphorothioate, and a 5'-mercapto moiety.

Synergistic compositions comprising the miRNA mimics compositions

The authors of the present invention have found that the combination of the miRNA mimic combination of the invention and SN38, which is a topoisomerase inhibitor, results in a synergistic effect in terms of the reduction in the viability of cells derived from a uveal melanoma line.

Accordingly, in another aspect, the invention relates to a composition comprising:

- a miRNA mimic combination according to the invention and
- a chemotherapeutic agent

The term chemotherapeutic compound, as used herein, refers to a therapeutic agent known to be of use in chemotherapy for cancer. Such diseases include tumors, neoplasms, and cancer as well as diseases characterized by hyperplastic growth. In one embodiment, a chemotherapeutic agent is an agent of use in treating neoplasms such as solid tumors. In one embodiment, a chemotherapeutic agent is radioactive molecule.

Chemotherapeutic agents include those known by those skilled in the art, including but not limited to: 5-fluorouracil (5-FU), azathioprine, cyclophosphamide, antimetabolites (such as Fludarabine), antineoplastics (such as Etoposide, Doxorubicin, methotrexate, and Vincristine), carboplatin, cis- platinum and the taxanes, such as taxol. Rapamycin has also been used as a chemotherapeutic.

In one embodiment, the chemotherapeutic agent is a topoisomerase inhibitor.

The term "topoisomerase inhibitor", as used herein, refers to agents designed to interfere with the action of

topoisomerase enzymes (topoisomerase I and II), which are enzymes that control the changes in DNA structure by catalyzing the breaking and rejoining of the phosphodiester backbone of DNA strands during the normal cell cycle. Topoisomerase inhibitors are often divided according to which type of enzyme it inhibits. Topoisomerase I inhibitors include, but are not limited to, irinotecan, topotecan, camptothecin, and lamellarin D, and all target type IB topoisomerases. Topoisomerase II inhibitors include, but are not limited to, etoposide (VP-16), teniposide, doxorubicin, daunorubicin, mitoxantrone, amsacrine, ellipticines, aurintricarboxylic acid, and HU-331, a quinolone synthesized from cannabidiol. In some embodiments of the methods and compositions disclosed herein, the topoisomerase inhibitor is a topoisomerase I inhibitor (e.g., irinotecan, nanoliposomal irinotecan, topotecan, camptothecin, indotecan, indimitecan, or SN-38).

In one embodiment, the topoisomerase inhibitor is a topoisomerase I inhibitor. In a preferred embodiment, the topoisomerase I inhibitor is SN38 (7-ethyl-10-hidroxycamptothecin)

In another embodiment, the topoisomerase inhibitor is a topoisomerase II inhibitor. The term "topoisomerase II inhibitor" as used herein includes, but is not limited to the anthracyclines such as doxorubicin (including liposomal formulation, such as Caelyx™), daunorubicin, epirubicin, idarubicin and nemorubicin, the anthraquinones mitoxantrone and losoxantrone, and the podophillotoxines etoposide and teniposide. Etoposide is marketed under the trade name Etopophos™. Teniposide is marketed under the trade name VM 26-Bristol Doxorubicin is marketed under the trade name Acriblastin™ or Adriamycin™. Epirubicin is marketed under the trade name Farmorubicin™. Idarubicin is marketed. under the trade name Zavedos™. Mitoxantrone is marketed under the trade name Novantron.

The compositions according to the present invention comprising the DNA mimics combination (first component of the synergistic composition) and the chemotherapeutic agent (second component of the synergistic composition) show a synergistic behavior.

The term "synergistic" refers to a combination which is more effective than the additive effects of any two or more single therapeutic agents. A synergistic effect permits the effective treatment of a disease, disorder or condition using lower amounts (doses) of individual therapeutics. The lower doses may result in lower toxicity (and/or reduced adverse events or severity of adverse events) without reduced efficacy. Alternatively, a synergistic effect can result in improved efficacy. Alternatively still, synergy may result in an improved avoidance or reduction of disease as compared to any single therapy.

Combination therapy or co-therapy can allow for the product of lower doses of the first therapeutic or the second therapeutic agent (referred to as "apparent one-way synergy" herein), or lower doses of both therapeutic agents (referred to as "two-way synergy" herein) than would normally be required when either drug is used alone. In certain embodiments, the synergism exhibited between one or more therapeutic agent(s) and the remaining therapeutic agent(s) is such that the dosage of one of the therapeutic agents would be sub-therapeutic if administered without the dosage of the other therapeutic agent(s).

The terms "augmentation" or "augment" refer to combinations where one of the compounds increases or enhances therapeutic effects of another compound or compounds administered to a patient. In some instances, augmentation can result in improving the efficacy, tolerability, or safety, or any combination thereof, of a particular therapy.

In certain embodiments, the present invention is directed in part to synergistic combinations of in an amount sufficient to render a therapeutic effect together with the remaining therapeutic agent(s). For example, in certain embodiments a therapeutic effect is attained which is at least about 2 (or at least about 4, 6, 8, or 10) times greater than that obtained with the dose of the one or more therapeutic agent(s) alone. In certain embodiments, the synergistic combination provides a therapeutic effect which is up to about 20, 30 or 40 times greater than that obtained with the dose of the one or more therapeutic agent(s) alone. In such embodiments, the synergistic combinations display what is referred to herein as an "apparent one-way synergy", meaning that the dose of the remaining therapeutic agent(s) synergistically potentiates the effect of the one or more therapeutic agent(s), but the dose of the one or more therapeutic agent(s) does not appear to significantly potentiate the effect of the remaining therapeutic agent(s).

In certain embodiments, the combination of active agents exhibits two-way synergism, meaning that the second therapeutic agent potentiates the effect of the first therapeutic agent, and the first therapeutic agent potentiates the effect of the second therapeutic agent. Thus, other embodiments of the invention relate to combinations of a second therapeutic agent and a first therapeutic agent where the dose of each drug is reduced due to the synergism between the drugs, and the therapeutic effect derived from the combination of drugs in reduced doses is enhanced. The two-way synergism is not always readily apparent in actual dosages due to the potency ratio of the first therapeutic agent to the second therapeutic agent. For instance, two-way synergism can be difficult to detect when one therapeutic agent displays much greater therapeutic potency relative to the other therapeutic agent.

In co-therapy, the miRNA mimic combination according to the invention may be included in the same pharmaceutical composition as the chemotherapeutic agent, or the miRNA mimic combination may be included in a separate pharmaceutical composition. In this latter case, the miRNA mimic combination is suitable for administration prior to, simultaneously with, or following administration of the pharmaceutical composition comprising the chemotherapeutic agent. In certain instances, the administration of the miRNA mimic combination to a subject in one composition overlaps with the time of administration of the chemotherapeutic agent in a separate composition.

In a preferred embodiment, the miRNA mimic combination according to the invention and the chemotherapeutic agent are provided in the same pharmaceutical composition.

Nanoparticles

The authors of the present invention have also found that the internalization, stability and/or solubility of the therapeutic molecules forming part of the combinations and compositions according to the present invention can be improved when they are attached to nanoparticles acting as carriers.

Accordingly, in another aspect, the invention relates to a composition wherein one or more of the miRNA mimics according to the invention are attached to a nanoparticle. In another aspect, the invention relates to a composition comprising a miRNA mimic composition and a chemotherapeutic compound wherein the chemotherapeutic compound is covalently coupled to a nanoparticle. In another aspect, the invention relates to a composition comprising a miRNA mimic composition and a chemotherapeutic compound wherein one or more of the miRNA mimics and the chemotherapeutic compound are covalently coupled to a nanoparticle.

The term "nanoparticle" as used herein is intended to refer to particles that have at least one dimension that is less than one micrometer.

The nanoparticle ranges in size from 5 to 300 nm in diameter, e.g., 20 to 280, 30 to 250, 40 to 200, 20 to 150, 30 to 100, 20 to 80, 30 to 70, 40 to 60 or 40 to 50 nm diameter. In one embodiment, the particle is 50 to 60 nm, 20 to 60 nm, 30 to 60 nm, 35 to 55 nm, 35 to 50 nm or 35 to 45 nm in diameter. In a preferred embodiment, the nanoparticles have an average size of 13-15 nm.

In one embodiment, the composition comprising nanoparticles contains only the miRNA mimics and not a chemotherapeutic agent. In this case, one or more of the miRNA mimics can be attached to the nanoparticle. In one embodiment, only the miRNA-34a mimic is attached to the nanoparticle, whereas the other miRNA mimics are provided in the free form, i.e. not conjugated to a nanoparticle. In one embodiment, only the miRNA-137 mimic is attached to the nanoparticle, whereas the other miRNA mimics are provided in free form, i.e. not conjugated to a nanoparticle. In one embodiment, only the miRNA-182 mimic is attached to the nanoparticle, whereas the other miRNA mimics are provided in free form, i.e. not conjugated to a nanoparticle. In one embodiment, only the miRNA-144 mimic is attached to the nanoparticle, whereas the other miRNA mimics are provided in free form, i.e. not conjugated to a nanoparticle

In another embodiment, two of the miRNA mimics are attached to the nanoparticle. Any preferred combination of two mimics can be attached to the nanoparticle, i.e. the miRNA-34a mimic and the miRNA-137 mimic, the miRNA-34a mimic and the miRNA-182 mimic,. the miRNA-34a mimic and the miRNA-144 mimic, the miRNA-137 mimic and the miRNA-182 mimic, the miRNA-137 mimic and the miRNA-144 mimic or the miRNA-182 mimic and the miRNA-144 mimic. In this case, both mimics can be provided in the same nanoparticles, i.e., the nanoparticles are coupled to two types of mimics or, alternatively, each type of mimic is attached to a nanoparticle independently of the other and thus, the compositions are used by combining the two types of nanoparticles each one carrying different miRNA mimics.

In another embodiment, three of the miRNA mimics are attached to the nanoparticle. Any preferred combination of three mimics can be attached to the nanoparticle, i.e. the miRNA-34a mimic, the miRNA-137 mimic and the miRNA-182 mimic, the miRNA-34a mimic, the miRNA-137 mimic and the miRNA-144 mimic, the miRNA-137 mimic, the miRNA-182 mimic and the miRNA-144 mimic. In this case, the three mimics can be provided in the same nanoparticles, i.e., the nanoparticles are coupled to three types of mimics or, alternatively, each type of mimic is attached to a nanoparticle independently of the other and thus, the compositions are used by combining the three types of nanoparticles each one carrying different miRNA mimics.

In another embodiment, the four miRNA mimics (the miRNA-34a mimic, the miRNA-137 mimic, the miRNA-182 mimic and the miRNA-144 mimic) are attached to nanoparticles. In this case, the four mimics can be provided in the same nanoparticles, i.e., the nanoparticles are coupled to the four types of mimics or, alternatively, each type of mimic is attached to a nanoparticle independently of the other and thus, the compositions are used by combining the four types of nanoparticles each one carrying different miRNA mimics.

In a preferred embodiment, a single type of nanoparticle contains the miRNA-34a mimic, the miRNA-137 mimic, the miRNA-182 mimic and the miRNA-144 mimic.

In another aspect, the invention relates to a composition comprising a miRNA mimic composition and a chemotherapeutic compound wherein the chemotherapeutic compound is covalently coupled to a nanoparticle.

In another aspect, the invention relates to a composition comprising a miRNA mimic composition wherein one or more of the mimics are attached to a nanoparticle and wherein the chemotherapeutic agent is attached to the nanoparticle. The composition may contain at least two types of nanoparticles, one of them modified with one or more miRNA mimics and the other one modified with the chemotherapeutic agent. However, in a preferred embodiment, the composition is formed by nanoparticles that contain the four miRNA mimics of the composition according to the invention as well as the chemotherapeutic agent.

Nanoparticles useful in the practice of the invention include metal (e.g., gold, silver, copper and platinum),

semiconductor (e.g., CdSe, CdS, and CdS or CdSe coated with ZnS) and magnetic (e.g., ferromagnetite) colloidal materials. Other nanoparticles useful in the practice of the invention include ZnS, ZnO, $TiO_2$, AgI, AgBr, $HgI_2$, PbS, PbSe, ZnTe, CdTe, $In_2S_3$, $In_2Se_3$, $Cd_3P_2$, $Cd_3As_2$, InAs, and GaAs. In a preferred embodiment, the nanoparticles are gold nanoparticles (AuNPs).

It will be understood that the miRNA mimics can be bound to the nanoparticle through any means. Regardless of the means by which the oligonucleotide is attached to the nanoparticle, attachment in various aspects is effected through a 5' linkage, a 3' linkage, some type of internal linkage, or any combination of these attachments. In the case of DNA-based miRNA mimics, the oligonucleotides may be attached by their 5' end or by their 3' end. In the case of RNA-based miRNA mimics, they can be attached to the nanoparticles by the 5' end of the guide strand, by the 3' end of the guide strand, by the 5' end of the passenger strand or by the 3' end of the passenger strand. It will also be understood that not all nucleic acids must be attached to the nanoparticles by the same end and that the composition may comprise nanoparticles wherein some of the nucleic acids are attached by the 5' end and some of the nucleic acids are attached by the 3' end.

In one embodiment, the miRNA mimics are mimics are covalently coupled to a nanoparticle.

In a preferred embodiment, the miRNA mimics are covalently coupled to the nanoparticle by a disulfide bond, the miRNA mimics comprises the sequences SEQ ID NO: 9, 11, 13 or 15 or a functionally variant equivalent thereof and the coupling is formed by the disulfide bond between the 5' end of said sequences and the nanoparticle.

In another preferred embodiment, the miRNA mimics are covalently coupled to the nanoparticle by a disulfide bond, the miRNA mimics comprises the sequences SEQ ID NO: 1-4 or a functionally variant equivalent thereof and the coupling is formed by the disulfide bond between the 3' end of said sequences and the nanoparticle.

In another embodiment, the combination according to the invention contains two types of nanoparticles: a first type comprising the four miRNA mimics and a second one containing the chemotherapeutic agent.

In one embodiment, nanoparticles may be modified with hydrophilic polymers such as polyethylene glycol (PEG; PEGylated NPs) or pluronics to sustain their time in the circulation.

In a preferred embodiment, the nanoparticles may further comprise a moiety which is capable of specific delivery of the nanoparticles to the cells of interest. This moiety can be attached to the nanoparticles containing the miRNA mimics, to the nanoparticles containing the chemotherapeutic agent or to both types of nanoparticles. In a preferred embodiment, the moiety capable of delivering the nanoparticle to cancer cells is a moiety having affinity towards nucleolin. In a more preferred embodiment, the moiety is an aptamer. In a still more preferred embodiment, the aptamer is comprises the sequence shown in SEQ ID NO: 25. Preferably, the aptamer is covalently bound to the nanoparticle by a disulfide bond, said disulfide bond present at the 3' end of said sequence.

Methods of making nanoparticles of the invention are well-known in the art and usually comprise contacting an activated form of the nucleic acid that contains a reactive group and an activated form of the nanoparticle which contains a group which is capable of reacting with the reactive group found in the nucleic acid which leads to the formation of a linker which connects the nucleic acid and the nanoparticle. Such methods are known in the art. For instance, oligonucleotides functionalized with alkanethiols at their 3'-termini or 5'-termini readily attach to gold nanoparticles [R. Elghanian, J.J. Storhoff, R.C. Mucic, R.L. Letsinger and C.A. Mirkin, Selective colorimetric detection of polynucleotides based on the distance-dependent optical properties of gold nanoparticles. Science 277 (1997), pp. 1078-1081].V See also, Mucic et al. Chem. Commun. 555-557 (1996) (describes a method of attaching 3' thiol DNA to flat gold surfaces; this method can be used to attach oligonucleotides to nanoparticles). The alkanethiol method can also be used to attach oligonucleotides to other metal, semiconductor and magnetic colloids and to the other nanoparticles listed above. Other functional groups for attaching oligonucleotides to solid surfaces include phosphorothioate groups, substituted alkylsiloxanes for binding of oligonucleotides to silica and glass surfaces, and for similar binding of mercaptoaklylsiloxanes. A cyclic dithiane-epiandrosterone disulfide linker has been developed for binding oligonucleotides to gold surfaces [R. Elghanian, J.J. Storhoff, R.C. Mucic, R.L. Letsinger and C.A. Mirkin, Selective colorimetric detection of polynucleotides based on the distance-dependent optical properties of gold nanoparticles. Science 277 (1997), pp. 1078-1081]. Li et al. have reported a trithiol-capped oligonucleotide that can stabilize gold metal nanoparticles having diameters greater than or equal to 100 nm, while retaining hybridization properties that are comparable to acyclic or dithiololigonucleotide modified particles [Z. Li, R.C. Jin, C.A. Mirkin and R.L. Letsinger, Multiple thiol-anchor capped DNA-gold nanoparticle conjugates. Nucleic Acids Res. 30 (2002), pp. 1558-1562]. In another embodiment, the oligonucleotides are modified with a dihiol reactive group such as dithiol lipoic acid as it has been described that this type of reactive groups provide a very efficient coupling to nanoparticles and, in particular, to gold nanoparticles.

Oligonucleotides terminated with a 5' thionucleoside or a 3' thionucleoside may also be used for attaching oligonucleotides to solid surfaces.

In certain aspects wherein cyclic disulfide functionalization, oligonucleotides are attached to a nanoparticle through one or more linkers. In one embodiment, the linker comprises a hydrocarbon moiety attached to a cyclic disulfide. Suitable hydrocarbons are available commercially, and are attached to the cyclic disulfides. The hydrocarbon moiety is, in one aspect, a steroid residue. Oligonucleotide-nanoparticle conjugates prepared using linkers comprising a steroid

residue attached to a cyclic disulfide are more stable to thiols compared to conjugates prepared using alkanethiols or acyclic disulfides as the linker, and in certain instances, the oligonucleotide-nanoparticle conjugates have been found to be 300 times more stable. In certain embodiments, the two sulfur atoms of the cyclic disulfide are close enough together so that both of the sulfur atoms attach simultaneously to the nanoparticle. In other aspects, the two sulfur atoms are adjacent to each other. In aspects where utilized, the hydrocarbon moiety is large enough to present a hydrophobic surface screening the surfaces of the nanoparticle.

Each nanoparticle may have a plurality of oligonucleotides attached to it, which may be the same or different. As a result, each nanoparticle-oligonucleotide conjugate can bind to a plurality of oligonucleotides or nucleic acids having the complementary sequence.

The nucleic acids according to the present invention may be attached to the nanoparticle directly or by a linker or spacer region. The linker or spacer region may comprise about 4-40, about 4-30, about 4-20, about 4-15, about 7-50, about 7- 40, about 7-30, about 7-20 atoms, about 7-15, about 6-15, about 7-14, about 8-14, about 9-13 atoms. In one embodiment any of the atoms forming the linker are independently selected from carbon, nitrogen, oxygen, sulfur and silicon. In one embodiment the linker is covalently attached to the nanoparticle by a silicon or sulfur atom. Thus, any of the atoms of the linker are independently selected from carbon, nitrogen, oxygen, sulfur, silicon and hydrogen.

Therapeutic uses of the combinations and nanoparticles

The authors of the present invention have observed that the miRNA mimics combinations as described in the present invention are useful in reducing the viability of tumor cells. This effect may be enhanced synergistically when the combinations are administered together with a chemotherapeutic agent.

Accordingly, in another aspect, the invention relates to the miRNA mimic combination according to the invention for use in medicine. In another aspect, the invention relates to a composition comprising the miRNA mimic combination according to the invention and a chemotherapeutic agent for use in medicine. In another aspect, the invention relates to the miRNA mimic combination according to the invention for use in the treatment and/or prevention of cancer. In another aspect, the invention relates to a composition comprising the miRNA mimic combination according to the invention and a chemotherapeutic agent for use in the treatment of cancer.

The term miRNA mimic combination as used herein has been described in detail above and the different embodiments are equally applicable to the therapeutic methods described herein. The miRNA mimic combinations can be used in therapy in free form, i.e. not attached to a support or, alternatively, as nanoparticles containing one or more of the miRNA mimics. In a preferred embodiment, the miRNA mimics are used in therapy as nanoparticles which comprise the four miRNA mimics. In a more preferred embodiment, a single type of nanoparticle is used which contains the four miRNA mimics. In a preferred embodiment, the miRNA mimics forming part of the nanoparticle are RNA-based miRNA mimics.

The term cancer, as used herein, refers to any disease caused by the proliferation of neoplastic cells, such as solid tumors, neoplasms, carcinomas, sarcomas, leukemias, lymphomas and the like. The term "cancer" includes primary malignant cells or tumors (e.g., those whose cells have not migrated to sites in the subject's body other than the site of the original malignancy or tumor) and secondary malignant cells or tumors (e.g., those arising from metastasis, the migration of malignant cells or tumor cells to secondary sites that are different from the site of the original tumor). In a preferred embodiment, the cancer to the treated is a primary tumor. In another embodiment, the cancer to the treated is a metastasis.

The method can reduce tumor size or burden, or prevent tumor growth compared to, for example, an untreated control. In some embodiments, the combinations and compositions according to the present invention are utilized to treat the cancer directly by delivering a therapeutic active agent to, or preferably into the cytosol of a cancer cell.).

The types of cancer that may be treated with the provided compositions and methods include, but are not limited to, the following: bladder, brain, breast, cervical, colo-rectal, esophageal, kidney, liver, lung, nasopharyngeal, pancreatic, prostate, skin, stomach, uterine, ovarian, testicular and hematologic. In a preferred embodiment, the cancer to be treated is uveal melanoma.

The term "uveal melanoma", as used herein, refer to a tumor caused by the uncontrolled proliferation of the pigment cells (melanocytes) that reside within the uvea giving color to the eye and which usually involves the iris, the ciliary body, or the choroid (collectively referred to as the uvea).

In particular embodiment, the combination results from the simultaneous administration of each of the components wherein each component is provided separately. In a further particular embodiment, the combination results from a sequential administration of all the components, wherein the components are administered at different time points. In another particular embodiment, the combination consists on a formulation comprising all the components of the combination, preferably on a medicament comprising all the components of the combination.

The combination can result from a combination of combinations. Thus, in a preferred embodiment, at least 2, preferably at least 3 of the components of the combination are administered simultaneously, wherein each component

is provided separately, and at least 2, preferably at least 1 of the rest of components are administered separately at a different time point. In another preferred embodiment, at least 2, or at least 3 of the components of the combination are administered as a formulation, preferably as a medicament and at least 1 or at least 2 of the rest of components are administered separately but simultaneously.

In another preferred embodiment, at least 2, or at least 3 of the components of the combination are administered as a formulation, preferably as a medicament and at least 1 or at least 2 of the rest of components are administered separately at a different time point.

The invention also encompasses the use of a pharmaceutical composition of the invention to practice the methods of the invention. Such a pharmaceutical composition may be provided in a form suitable for administration to a subject, and may be comprise one or more pharmaceutically acceptable carriers, one or more additional ingredients, or some combination of these. The at least one composition of the invention may comprise a physiologically acceptable salt, such as a compound contemplated within the invention in combination with a physiologically acceptable cation or anion, as is well known in the art.

Pharmaceutical compositions that are useful in the methods of the invention may be suitably developed for inhalational, oral, rectal, vaginal, parenteral, topical, transdermal, pulmonary, intranasal, buccal, ophthalmic, intrathecal, intravenous or another route of administration. Other contemplated formulations include projected nanoparticles, liposomal preparations, resealed erythrocytes containing the active ingredient, and immunologically-based formulations. The route(s) of administration will be readily apparent to the skilled artisan and will depend upon any number of factors including the type and severity of the disease being treated, the type and age of the veterinary or human patient being treated, and the like.

The formulations of the pharmaceutical compositions described herein may be prepared by any method known or hereafter developed in the art of pharmacology. In general, such preparatory methods include the step of bringing the active ingredient into association with a carrier or one or more other accessory ingredients, and then, if necessary or desirable, shaping or packaging the product into a desired single- or multidose unit.

In one embodiment, the compositions of the invention are formulated using one or more pharmaceutically acceptable excipients or carriers. In one embodiment, the pharmaceutical compositions of the invention comprise a therapeutically effective amount of at least one compound of the invention and a pharmaceutically acceptable carrier. Pharmaceutically acceptable carriers, which are useful, include, but are not limited to, glycerol, water, saline, ethanol and other pharmaceutically acceptable salt solutions such as phosphates and salts of organic acids. Examples of these and other pharmaceutically acceptable carriers are described in Remington's Pharmaceutical Sciences (1991, Mack Publication Co., New Jersey).

When used in therapy, the combinations and compositions according to the present invention will have to be administered to a subject in need thereof. It will be understood that the administration will have to be adapted depending on whether the patient is to be treated with the miRNA mimics combination according to the present invention or whether the patient is to be treated with nanoparticles containing the miRNA mimics according to the invention.

*Nucleic acid delivery*

The delivery of nucleic acids (DNA-based miRNA mimics, RNA-based miRNA mimics or polynucleotides encoding miRNAs) can be carried out using non-viral means involving transfection in vitro. Such methods include the use of calcium phosphate, DEAE dextran, electroporation, and protoplast fusion. Liposomes can also be potentially beneficial for delivery of DNA into a cell. In other embodiments, inhibitory nucleic acids of the invention can be delivered without transfection or electroporation.

The term "transfection" as used herein means an introduction of a foreign DNA or RNA into a cell by mechanical inoculation, electroporation, infection, particle bombardment, microinjection, or by other known methods. Alternatively, one or a combination of expression vectors can be used to transform the cells and cell lines. The term "transformation" as used herein means a stable incorporation of a foreign DNA or RNA into the cell which results in a permanent, heritable alteration in the cell. A variety of suitable methods are known in the field and have been described. See e.g., Ausubel et al., supra; Sambrook, supra; and the Promega Technical Manual.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al., MOLECULAR CLONING: A LABORATORY MANUAL volumes 1-3 (3rd ed., Cold Spring Harbor Press, N Y 2001).

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. See, for example, U.S. Pat. Nos. 5,350,674 and 5,585,362.

Chemical means for introducing a polynucleotide into a host cell include colloidal dispersion systems, such as

macromolecule complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g., an artificial membrane vesicle).

In the case where a non-viral delivery system is utilized, an exemplary delivery vehicle is a liposome. The use of lipid formulations is contemplated for the introduction of the nucleic acids into a host cell (in vitro, ex vivo or in vivo). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

Lipids suitable for use can be obtained from commercial sources. For example, dimyristyl phosphatidylcholine ("DMPC") can be obtained from Sigma, St. Louis, Mo.; dicetyl phosphate ("DCP") can be obtained from K and K Laboratories (Plainview, N.Y.); cholesterol ("Choi") can be obtained from Calbiochem-Behring; dimyristyl phosphatidylglycerol ("DMPG") and other lipids may be obtained from Avanti Polar Lipids, Inc. (Birmingham, Ala.). Stock solutions of lipids in chloroform or chloroform/methanol can be stored at about -20 degrees centigrade Chloroform is used as the only solvent since it is more readily evaporated than methanol. "Liposome" is a generic term encompassing a variety of single and multilamellar lipid vehicles formed by the generation of enclosed lipid bilayers or aggregates. Liposomes can be characterized as having vesicular structures with a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh et al., 1991 Glycobiology 5: 505-10). However, compositions that have different structures in solution than the normal vesicular structure are also encompassed. For example, the lipids may assume a micellar structure or merely exist as nonuniform aggregates of lipid molecules. Also contemplated are lipofectamine-nucleic acid complexes.

In the case of the delivery of nucleic acids encoding a miRNA or of a combination thereof, the nucleic acids are usually expressed from a larger polynucleotide which contains a suitable promoter which is operably linked with the nucleic acid.. Alternatively, regulation can be mediated by cognate regulatory sequences or, if desired, by regulatory sequences derived from a heterologous source, including any of the promoters or regulatory elements described above.

The nucleic acid can be cloned into a number of types of vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art. Viruses, which are useful as vectors include, but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers.

In the clinical settings, delivery systems for the therapeutic composition can be introduced into a patient by any of a number of methods, each of which is familiar in the art. For instance, a pharmaceutical composition can be introduced systemically, e.g. by intravenous injection, and specific transduction of the protein in the target cells occurs predominantly from specificity of transfection provided by the gene delivery vehicle, cell-type or tissue-type expression due to the transcriptional regulatory sequences controlling expression of the receptor gene, or a combination thereof. In other embodiments, initial delivery of the recombinant gene is more limited with introduction into the animal being quite localized. For example, the gene delivery vehicle can be introduced by catheter (see U.S. Pat. No. 5,328,470) or by stereotactic injection (e.g. Chen, et al. PNAS 91: 3054-3057 (1994)). The preparation may also be provided to cells ex vivo. Cells containing the miRNAs (e.g., miR-424 and/or miR-503) are then administered to the patient.

The regimen of administration may affect what constitutes an effective amount. The therapeutic formulations may be administered to the patient either prior to or after the manifestation of symptoms associated with the disease or condition. Further, several divided dosages, as well as staggered dosages may be administered daily or sequentially, or the dose may be continuously infused, or maybe a bolus injection. Further, the dosages of the therapeutic formulations may be proportionally increased or decreased as indicated by the exigencies of the therapeutic or prophylactic situation.

Administration of the compositions of the present invention to a patient, preferably a mammal, more preferably

a human, may be carried out using known procedures, at dosages and for periods of time effective to treat a disease or condition in the patient. An effective amount of the therapeutic compound necessary to achieve a therapeutic effect may vary according to factors such as the activity of the particular compound employed; the time of administration; the rate of excretion of the compound; the duration of the treatment; other drugs, compounds or materials used in combination with the compound; the state of the disease or disorder, age, sex, weight, condition, general health and prior medical history of the patient being treated, and like factors well-known in the medical arts. Dosage regimens may be adjusted to provide the optimum therapeutic response. For example, several divided doses may be administered daily or the dose may be proportionally reduced as indicated by the exigencies of the therapeutic situation. A non-limiting example of an effective dose range for a therapeutic compound of the invention is from about 0.01 and 50 mg/kg of body mass/per day. One of ordinary skill in the art would be able to study the relevant factors and make the determination regarding the effective amount of the therapeutic compound without undue experimentation.

Human dosage amounts can initially be determined by extrapolating from the amount of compound used in mice, as a skilled artisan recognizes it is routine in the art to modify the dosage for humans compared to animal models. In certain embodiments it is envisioned that the dosage may vary from between about 1 $\mu$g compound/Kg body mass to about 5000 mg compound/kg body mass; or from about 5 mg/kg body mass to about 4000 mg/kg body mass or from about 10 mg/kg body mass to about 3000 mg/kg body mass; or from about 50 mg/kg body mass to about 2000 mg/kg body mass; or from about 100 mg/kg body mass to about 1000 mg/kg body mass; or from about 150 mg/kg body mass to about 500 mg/kg body mass. In other embodiments this dose may be about 1, 5, 10, 25, 50, 75, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1600, 1700, 1800, 1900, 2000, 2500, 3000, 3500, 4000, 4500, or 5000 mg/kg body mass. In other embodiments, it is envisaged that doses may be in the range of about 5 mg compound/kg body to about 20 mg compound/kg body mass. In other embodiments the doses may be about 8, 10, 12, 14, 16 or 18 mg/kg body mass. Of course, this dosage amount may be adjusted upward or downward, as is routinely done in such treatment protocols, depending on the results of the initial clinical trials and the needs of a particular patient.

Actual dosage levels of the active ingredients in the pharmaceutical compositions of this invention may be varied so as to obtain an amount of the active ingredient that is effective to achieve the desired therapeutic response for a particular patient, composition, and mode of administration, without being toxic to the patient.

In one embodiment, the present invention is directed to a packaged pharmaceutical composition comprising a container holding a therapeutically effective amount of a compound of the invention, alone or in combination with a second pharmaceutical agent; and instructions for using the compound to treat, prevent, or reduce one or more symptoms of a disease or disorder in a patient.

Routes of administration of any of the compositions of the invention include inhalational, oral, nasal, rectal, parenteral, sublingual, transdermal, transmucosal (e.g., sublingual, lingual, (trans)buccal, (trans)urethral, vaginal (e.g., trans- and perivaginally), (intra)nasal, and (trans)rectal), intravesical, intrapulmonary, intraduodenal, intragastrical, intrathecal, subcutaneous, intramuscular, intradermal, intra-arterial, intravenous, intrabronchial, inhalation, and topical administration.

Suitable compositions and dosage forms include, for example, tablets, capsules, caplets, pills, gel caps, troches, dispersions, suspensions, solutions, syrups, granules, beads, transdermal patches, gels, powders, pellets, magmas, lozenges, creams, pastes, plasters, lotions, discs, suppositories, liquid sprays for nasal or oral administration, dry powder or aerosolized formulations for inhalation, compositions and formulations for intravesical administration and the like. It should be understood that the formulations and compositions that would be useful in the present invention are not limited to the particular formulations and compositions that are described herein.

The compositions according to the present invention (free miRNA mimic combinations or nanoparticles onto which the miRNA mimics and/or the chemotherapeutic agent are attached) may be administered to different anatomical areas of the eye. For example, the compositions may be administered by injection into the vitreous body just outside of the lens, into the center of the vitreous body, on top of the retina, in the subconjunctival space, in subretinal space, or on top of the optic nerve.

## Use of the combinations as adjuvant therapy in cancer treatment

In another aspect, the invention relates to the miRNA mimics combination according to the invention for use as an adjuvant in the treatment of cancer with a chemotherapeutic compound.

The methods may be practiced in an adjuvant setting. "Adjuvant setting" refers to a clinical setting in which, for example, an individual has had a history of a proliferative disease, particularly cancer, and generally (but not necessarily) been responsive to therapy, which includes, but is not limited to, surgery (such as surgical resection), radiotherapy, and chemotherapy. However, because of their history of the proliferative disease (such as cancer), these individuals are considered at risk of development of the disease. Treatment or administration in the "adjuvant setting" refers to a subsequent mode of treatment. The degree of risk (i.e., when an individual in the adjuvant setting is considered as "high

risk" or "low risk") depends upon several factors, most usually the extent of disease when first treated. The methods provided herein may also be practiced in a neoadjuvant setting, i.e., the method may be carried out before the primary/definitive therapy. Thus, in some embodiments, the individual has previously been treated. In other embodiments, the individual has not previously been treated. In some embodiments, the treatment is a first line therapy.

The invention is also defined by the following aspects:

1-A combination comprising a miRNA-34a mimic, a miRNA-182 mimic, a miRNA-137 mimic and a miRNA-144 mimic.

2- The combination according to aspect 1 wherein the miRNA-34a mimic, the miRNA-182 mimic, the miRNA-137 and the miRNA-144 mimic are independently selected from the group consisting of:

(i) a DNA oligonucleotide comprising a DNA version of the sequence of the mature miRNA or a functionally variant equivalent thereof,
(ii) a double-stranded RNA, wherein the sequence of one of the strands comprises the sequence of the mature miRNA or a functionally variant equivalent thereof and
(iii) a polynucleotide encoding the miRNA.

3- The combination according to aspect 2 wherein the miRNA mimics are DNA antisense oligonucleotides and wherein the miRNA-34a mimic comprises the DNA version of the sequence of the mature miRNA-34a as defined in the miRbase database under accession number MIMAT0000255 or a functionally variant equivalent thereof, the miRNA-182 mimic comprises the DNA version of the sequence of the mature miRNA-182 as defined in the miRbase database under accession number MIMAT0000259 or a functionally variant equivalent thereof, the miRNA-137 mimic comprises the DNA version of the sequence of the mature miRNA-137 as defined in the miRbase database under accession number MIMAT0000429 or a functionally variant equivalent thereof and/or wherein the miRNA-144 mimic comprises the DNA version of the sequence of the mature miRNA-144 as defined in the miRbase database under accession number MIMAT0004600 or a functionally variant equivalent thereof.

4- The combination according to aspect 3, wherein the miRNA-34a mimic comprises a sequence shown in SEQ ID NO: 1 or a functionally variant equivalent thereof, the miRNA-182 mimic comprises a sequence shown in SEQ ID NO: 2 or a functionally variant equivalent thereof, the miRNA-137 mimic comprises a sequence shown in SEQ ID NO: 3 or a functionally variant equivalent thereof and wherein the miRNA-144 mimic comprises a sequence shown in SEQ ID NO: 4 or a functionally variant equivalent thereof.

5- The combination according to aspect 2 wherein the miRNA mimics are double-stranded inhibitory RNAs comprising two anticomplementary RNA sequence regions and wherein the miRNA34a mimic comprises a RNA sequence region corresponding to the miRNA-34a as defined in the miRbase database under accession number MIMAT0000255 or a functionally variant equivalent thereof, wherein the miRNA-182 mimic comprises a RNA sequence region corresponding to the miRNA-182 as defined in the miRbase database under accession number MIMAT0000259 or a functionally variant equivalent thereof, the miRNA-137 mimic comprises an RNA sequence region corresponding to the miRNA-137 as defined in the miRbase database under accession number MIMAT0000429 or a functionally variant equivalent thereof and/or wherein the miRNA-144 mimic comprises an RNA sequence region corresponding to the miRNA-144 as defined in the miRbase database under accession number MIMAT0004600 or a functionally variant equivalent thereof.

6- The combination according to aspect 5 wherein the miRNA34a mimic comprises a first sequence region comprising the sequence shown in SEQ ID NO: 17 or a functionally variant equivalent thereof and a second sequence region comprising the sequence shown in SEQ ID NO: 18 or a functionally variant equivalent thereof, the miRNA-182 mimic comprises a first sequence region comprising the sequence shown in SEQ ID NO: 19 or a functionally variant equivalent thereof and a second sequence region comprising the sequence shown in SEQ ID NO: 20 or a functionally variant equivalent thereof, the miRNA-137 mimic comprises a first sequence region comprising the sequence shown in SEQ ID NO: 21 or a functionally variant equivalent thereof and a second sequence region comprising the sequence shown in SEQ ID NO: 22 or a functionally variant equivalent thereof and wherein the miRNA-144 mimic comprises a first sequence region comprising the sequence shown in SEQ ID NO: 23 or a functionally variant equivalent thereof and a second sequence region comprising the sequence shown in SEQ ID NO: 24 or a functionally variant equivalent thereof.

7- The combination according to any of aspects 1 to 6 wherein the combination comprises equimolar quantities of each miRNA mimic.

8- The combination according to any of aspect 1 to 7 further comprising a chemotherapeutic compound.

9- The combination according to aspect 8 wherein the chemotherapeutic compound is a topoisomerase inhibitor.

10- The combination according to aspect 9 wherein the topoisomerase inhibitor is 7-ethyl-10-hidroxycamptothecin.

11- The combination according to any of aspects 1 to 10, wherein the miRNA mimics are covalently coupled to a nanoparticle.

12- A combination according to any of aspects 8 to 11, wherein the chemotherapeutic compound is covalently coupled to a nanoparticle.

13- The combination according to any of aspects 11 to 12, wherein the nanoparticles are gold nanoparticles.

14- The combination according to any of aspects 11 to 13 wherein the miRNA mimics are covalently coupled to the nanoparticle by a disulfide bond and wherein if the miRNA mimics comprises the sequences SEQ ID NO: 9, 11, 13 or 15 or a functionally variant equivalent thereof the disulfide bond is formed at the 5' end of said sequences and wherein if the miRNA mimics comprises the sequences SEQ ID NO: 1-4 or a functionally variant equivalent thereof the disulfide bond is formed at the 3' end of said sequences.

15-The combination according to any of aspects 12 to 14 wherein the chemotherapeutic compound present in a nanoparticle is functionalized with an oligonucleotide having the sequence shown in SEQ ID NO: 17, wherein said sequence is covalently bond to the nanoparticle by a disulfide bond, said disulfide bond present at the 3' end of said sequence.

16- The combination according to any of aspects 11 to 15 wherein the nanoparticles have an average size of 13-15 nm.

17- The combination according to any of aspects 1 to 16 for use in medicine.

18- The combination according to any of aspects 1 to 16 for use in the treatment and/or prevention of cancer.

19- The combination for use according to aspect 18 wherein the cancer is a primary tumor or a metastatic tumor.

20- The combination for use according to aspects 18 or 19, wherein the cancer is characterized by showing low levels of miRNA-34a, miRNA-182, miRNA-137 and miRNA-144.

21- The combination for use according to any of aspects 18 to 20 wherein the cancer is uveal melanoma.

22- The combination for use according to any of aspects 17 to 21 wherein the miRNA mimics are administered at the same time.

23- The combination according to any of aspects 1 to 7, 11,13,14 and 16 for use as an adjuvant in the treatment of cancer with a chemotherapeutic compound.

24- A miRNA-34a mimic

25. The miRNA-34a mimic according to aspect 24 wherein the mimic is selected from the group consisting of:

(i) a DNA oligonucleotide comprising a DNA version of the sequence of the mature miRNA or a functionally variant equivalent thereof,
(ii) a double-stranded RNA, wherein the sequence of one of the strands comprises the sequence of the mature miRNA or a functionally variant equivalent thereof and
(iii) a polynucleotide encoding the miRNA.

26- The miRNA-34a mimic according to aspect 25 wherein the mimic is a DNA antisense oligonucleotide which comprises the DNA version of the sequence of the mature miRNA-34a as defined in the miRbase database under accession number MIMAT0000255 or a functionally variant equivalent thereof.

27- The miRNA-34a mimic according to aspect 26 wherein the miRNA-34a mimic comprises a sequence shown in SEQ ID NO: 1, SEQ ID NO:9 or a functionally variant equivalent thereof.

28- The miRNA-34a mimic according to aspect 23 which is a double-stranded inhibitory RNAs comprising two anticomplementary RNA sequence regions which comprises an RNA sequence region corresponding to the miRNA-34a as defined in the miRbase database under accession number MIMAT0000255 or a functionally variant equivalent thereof.

29-The miRNA-34a mimic according to aspect 28 wherein the mimic comprises a first sequence region comprising the sequence shown in SEQ ID NO: 17 or a functionally variant equivalent thereof and a second sequence region comprising the sequence shown in SEQ ID NO: 18 or a functionally variant equivalent thereof.

30- A miRNA-182 mimic.

31. The miRNA-182 mimic according to aspect 30 wherein the mimic is selected from the group consisting of:

(i) a DNA oligonucleotide comprising a DNA version of the sequence of the mature miRNA or a functionally variant equivalent thereof,
(ii) a double-stranded RNA, wherein the sequence of one of the strands comprises the sequence of the mature miRNA or a functionally variant equivalent thereof and
(iii) a polynucleotide encoding the miRNA.

32- The miRNA-182 mimic according to aspect 31 wherein the mimic is a DNA antisense oligonucleotide which comprises the DNA version of the sequence of the MIMAT0000259 or a functionally variant equivalent thereof.

33.- The miRNA-182 mimic according to aspect 32 wherein the miRNA-182 mimic comprises a sequence shown in SEQ ID NO: 6, SEQ ID NO: 10 or a functionally variant equivalent thereof.

34- The miRNA-182 mimic according to aspect 31 which is a double-stranded inhibitory RNAs comprising two

anticomplementary RNA sequence regions which comprise an RNA sequence region corresponding to the miRNA-182 as defined in the miRbase database under accession number MIMAT0000259 or a functionally variant equivalent thereof.

35-The miRNA mimic according to aspect 34 wherein the mimic comprises a first sequence region comprising the sequence shown in SEQ ID NO: 19 or a functionally variant equivalent thereof and a second sequence region comprising the sequence shown in SEQ ID NO: 20 or a functionally variant equivalent thereof.

36- A miRNA-137 mimic

37- The miRNA-137 mimic according to aspect 36 wherein the mimic is selected from the group consisting of:

(i) a DNA oligonucleotide comprising a DNA version of the sequence of the mature miRNA or a functionally variant equivalent thereof,
(ii) a double-stranded RNA, wherein the sequence of one of the strands comprises the sequence of the mature miRNA or a functionally variant equivalent thereof and
(iii) a polynucleotide encoding the miRNA.

38 - The miRNA-137 mimic according to aspect 37 wherein the mimic is a DNA antisense oligonucleotide which comprises the DNA version of the sequence of the mature miRNA-137 as defined in the miRbase database under accession number MIMAT0000429 or a functionally variant equivalent thereof.

40- The miRNA-137 mimic according to aspect 38 wherein the miRNA-137 mimic comprises a sequence shown in SEQ ID NO: 7, SEQ ID NO: 1 1 or a functionally variant equivalent thereof.

41- The miRNA-137 mimic according to aspect 37 which is a double-stranded inhibitory RNAs comprising two anticomplementary RNA sequence regions which comprises a RNA sequence region corresponding to the miRNA-137 as defined in the miRbase database under accession number MIMAT0000429 or a functionally variant equivalent thereof.

42-The miRNA mimic according to aspect 34 wherein the mimic comprises a first sequence region comprising the sequence shown in SEQ ID NO: 21 or a functionally variant equivalent thereof and a second sequence region comprising the sequence shown in SEQ ID NO: 22 or a functionally variant equivalent thereof.

43- A miRNA-144 mimic

44- The miRNA-144 mimic according to aspect 43 wherein the mimic is selected from the group consisting of:

(i) a DNA oligonucleotide comprising a DNA version of the sequence of the mature miRNA or a functionally variant equivalent thereof,
(ii) a double-stranded RNA, wherein the sequence of one of the strands comprises the sequence of the mature miRNA or a functionally variant equivalent thereof and
(iii) a polynucleotide encoding the miRNA.

45 - The miRNA-144 mimic according to aspect 44 wherein the mimic is a DNA antisense oligonucleotide which comprises the DNA version of the sequence of the mature miRNA-137 as defined in the miRbase database under accession number MIMAT0004600 or a functionally variant equivalent thereof.

46- The miRNA-144 mimic according to aspect 45 wherein the miRNA-144 mimic comprises a sequence shown in SEQ ID NO: 8, SEQ ID NO: 12 or a functionally variant equivalent thereof.

47- The miRNA-144 mimic according to aspect 44 which is a double-stranded inhibitory RNAs comprising two anticomplementary RNA sequence regions which comprises an RNA sequence region corresponding to the miRNA-144 as defined in the miRbase database under accession number MIMAT0004600 or a functionally variant equivalent thereof.

48-The miRNA mimic according to aspect 47 wherein the mimic comprises a first sequence region comprising the sequence shown in SEQ ID NO: 23 or a functionally variant equivalent thereof and a second sequence region comprising the sequence shown in SEQ ID NO: 24 or a functionally variant equivalent thereof.

49- A miRNA mimic according to any of aspects 24 to 48 which is coupled to a nanoparticle.

50- A composition comprising a miRNA mimic according to any of aspects 24 to 49 further comprising a chemotherapeutic compound.

51- The composition according to aspect 50 wherein the chemotherapeutic compound is a topoisomerase inhibitor.

53- The composition according to aspect 51 wherein the topoisomerase inhibitor is 7-ethyl-10-hidroxycamptothecin.

54- The composition according to any of aspects 49 to 53, wherein the miRNA mimics are covalently coupled to a nanoparticle.

55- The composition according to any of aspects 51 to 54, wherein the chemotherapeutic compound is covalently coupled to a nanoparticle.

56- The composition according to any of aspects 49 to 55, wherein the nanoparticles are gold nanoparticles.

57- The composition according to any of aspects 49 to 56 for use in medicine.
58- The composition according to any of aspects 49 to 57 for use in the treatment and/or prevention of cancer.
59- The composition for use according to aspect 58 wherein the cancer is a primary tumor or a metastatic tumor.
60- The composition for use according to any of aspects 58 or 59 wherein the cancer is uveal melanoma.

The invention will be described by way of the following examples which are to be considered as merely illustrative and does not limitate of the scope of the invention.

## EXAMPLES

*Materials and methods*

Oligonucleotides (DNA and RNA) were obtained from IDT or prepared as described. The sequences of the oligonucleotides employed are described in Table 1. siRNA duplexes were obtained after annealing of complementary sequences using an annealing buffer described in "RNA annealing" section. The aptamer AS1411 was prepared as previously reported [Latorre, A. et al. Nanoscale 2014, 6, 7436].

Table 2: Oligonucleotides (DNA and RNA) used in the invention.

| SEQ ID NO: | Oligonucleotides | Sequence |
|---|---|---|
| 9 | DNA-34a | 5'-TGGCAGTGTCTTAGCTGGTTGTTA AAA A-3' |
| 10 | DNA-182 | 5' TTT GGC AAT GGT AGA ACT CAC ACT AAA AA 3' |
| 11 | DNA-137 | 5' TTA TTG CTT AAG AAT ACG CGT AGA AAA A 3' |
| 12 | DNA- 144 | 5' GGA TAT CAT CATATACTGTAAGAA AAA 3' |
| 13 | DNA-34a | 5'TGGCAGTGTCTTAGCTGGTTGTTA AAA A-Thiol 3' |
| 14 | DNA-182 | 5' TTT GGC AAT GGT AGA ACT CAC ACT AAA AA-Thiol 3' |
| 15 | DNA-137 | 5' TTA TTG CTT AAG AAT ACG CGT AGA AAA A-Thiol 3' |
| 16 | DNA- 144 | 5' GGA TAT CAT CATATACTGTAAGAA AAA-Thiol 3' |
| 17 | RNA-34a pass | 5' thiol-AAAAAArCrArArCrCrArGrCrUrArArGrArCrArCrUrGrCrCrU 3' |
| 18 | RNA- 34a guide | 5' rUrGrGrCrArGrUrGrUrCrUrUrArGrCrUrGrGrUrUrGrU 3' |
| 19 | RNA-182 pass | 5' Thiol -AAAAAArGrUrGrUrGrArGrUrUrCrUrArCrCrArUrUrGrCrCrArArA 3' |
| 20 | RNA- 182 guide | 5' rUrUrUrGrGrCrArArUrGrGrUrArGrArArCrUrCrArCrArCrU 3' |
| 21 | RNA- 137 pass | 5' Thiol AAAAACUACGCGUAUUCUUAAGCAAUAA 3' |
| 22 | RNA- 137 guide | 5' rUrUrArUrUrGrCrUrUrArArGrArArUrArCrGrCrGrUrArG 3' |
| 23 | RNA- 144 pass | 5' Thiol-AAAArCrUrUrArCrArGrUrArUrArUrGrArUrGrArUrArUrCrC 3' |
| 24 | RNA- 144 guide | 5' rGrGrArUrArUrCrArUrCrArUrArUrArCrUrGrUrArArG 3' |
| 25 | AS1411 [25] | 5' GGTGGTGGTGGTTGTGGTGGTGGTGGTTTTTT-dithiolane 3' |

*2.2 Oligonucleotide solution preparation*

RNA annealing (siRNAs)

siRNA duplexes were obtained by mixing the guide and the passenger oligonucleotides in an annealing buffer. The annealing buffer was prepared following Sigma-Aldrich' s protocol for annealing oligonucleotides [Protocol for Annealing Oligonucleotides Sigma-Aldrich Available online: https://www.sigmaaldrich.com/technical-documents/protocols/biology/annealing-oligos.html (accessed on Jul 4, 2018).].

siRNA-34a was obtained from oligonucleotides of SEQ ID NO: 17 and 18; siRNA-182 was obtained from SEQ ID NO: 19 and 20; siRNA-137 was obtained from SEQ ID NO: 21 and 22; siRNA- 144 was obtained from SEQ ID NO: 23 and 24 (Table 2).

*siRNA mix*

A sample containing the 4 siRNAs previously prepared was obtained by mixing equimolar quantities of each siRNA.

*DNA mix*

DNA mix-1 (34,04 μM final concentration) was obtained from equimolar amounts of oligonucleotides of SEQ ID NO: 9, 10, 11 and 12 (Table 2) in water. This mixture contains unmodified oligonucleotides.

DNA mix-2 (20 μM final concentration) was obtained from equimolar amounts of oligonucleotides of SEQ ID NO: 13, 14, 15 and 16 (Table 2) in water. This mixture contains thiol-modified 141 oligonucleotides and are used for the functionalization of AuNPs.

*2.3 Gold nanoparticles synthesis and Characterization*

The gold nanoparticles were prepared following standard reported procedures [Posch, C.; Latorre, A.; Crosby, M. B.; Celli, A.; Latorre, A.; Vujic, I.; Sanlorenzo, M.; Green, G. A.; Weier, 479 J.; Zekhtser, M.; Ma, J.; Monico, G.; Char, D. H.; Jusufbegovic, D.; Rappersberger, K.; Somoza, Á.; 480 Ortiz-Urda, S. Biomed. Microdevices 2015, 17, 15.]. The bare nanoparticles were modified with the different components using the conditions summarized in Table 2. After the addition of each reagent, the solution was incubated for 30 min. The procedure was done as follows: to 1 mL of a solution of AuNPs (10 nM) the reagents were added in the order and quantities detailed in Table 2. The last step implies the addition of NaCl (70 μL, 5M) and incubation at room temperature overnight. The particles were then centrifuged down (300 g) at 4 °C, the supernatant removed and resuspended in water. The process was repeated three times to remove the unbound material.

Table 3: AuNPs conditions

| Nanoparticle | Reagents used in the preparation |
|---|---|
| **AuNP DNA-mix** | 1 mL AuNP + 500 μl DNA mix 2 |
| **AuNP siRNA-mix** | 1 mL AuNP + 500 μl siRNA mix |
| **AuNP SN38** | 1 mL AuNP + 5 μl SN38 (2m M in DMSO)+ 4.06 μl AS1411 (492μM) |

The hydrodynamic size of the AuNPs was measured by Dynamic light scattering (DLS) on a Zeta Sizer Nano- ZS.

*2.4 Cell culture and viability studies*

Mel 202 cells were cultured in RPMI medium with 10 % fetal bovine serum (FBS), 1 % Streptavidin-Penicillin and 1 % L-Glutamine at 37 °C in an incubator. All the procedures were performed inside a laminar flow hood Telstar CV-30/70. Cells were grown in 24 wells plates (30000 cells).

*2.4.1 AlamarBlue assay.*

stock solution of Resazurin sodium salt (1 mg/ml) in PBS was diluted 1 % (v/v) in complete RPMI. After incubation 3 h with the cells, the fluorescence was measured in a plate reader Synergy H4 Hybrid reader (BioTEK). Excitation (Ex) 550 nm; Emission (Em) 590 nm.

*2.4.2 Oligonucleotide Transfection*

50 μl OptiMem was mixed with oligonucleotides (14, 15, 16, 17) or DNA mix 1. Then, it was mixed with 1 μl Lipofectamine 2000 in 50 μl optiMEM. The final mixture was incubated for 20 min and then 100 μl was added to cells. The final concentration of 14, 15, 16, 17 or DNA mix 1 was 140 nM. After 24 h the cells were washed 3 times with saline phosphate buffer (PBS) and complete medium was added.

*2.4.3 Chemotherapy*

SN38 stock solution was prepared at 100 μM in DMSO. Then different concentrations of SN38 (100 nM, 50 nM, 25 nM) were prepared in complete medium. It was incubated 24 h with the cells, then washed with PBS. After additional 24 h, the alamarBlue assay was carried out.

*2.4.4 Combination treatment*

In this case, the oligonucleotides were transfected first, and after 24 h SN38 was added and incubated for 24 h. Then, the cells were washed with PBS and the viability studied.

*2.4.5 Nanoparticles treatment*

A volume of 100 µL functionalized AuNPs were added in optiMEM (500 µL total volume). The cells were incubated for 24 h, washed with PBS and the viability assessed.

*2.5 C-Met immunofluorescence*

The immunofluorescence staining of c-Met was done as reported [Riemer, J.; Hoepken, H. H.; Czerwinska, H.; Robinson, S. R.; Dringen, R. Anal. Biochem. 2004, 331, 370-375.] using an anti-c-Met (Ab 1003) antibody (Ab) 1: 200 as a primary antibody and a Goat anti-rabbit IgG Alexa 488 1: 200 as secondary. The nucleus staining was done with Hoechst 33342.

Fluorescence microscopy was done with 274.29 exposures units in a Leica Microscopy DMI3000 M, and the images were analyzed using a personalized script in Fiji program [del Moral, Á. Cell Fluorescence Weber Available online: https://github.com/DelmoPy/Cell-Fluorescence-Weber.] .

*2.6 Flow cytometry*

The samples were trypsinized (BioWest) and fixed with paraformaldehyde 1 % (v/v). The samples were stained for 3 h with 10 µg/sample RNAsa A from Thermo Scientific and 20 µg/sample propidium iodide from Sigma. The cell cycle analysis was performed in a Beckman coulter Cytomics 500 Flow cytometer and acquired data were analyzed with the Multicycle software.

These experiments were performed in cytometry service of Biotechnology National Centre of Spain.

*2.7 Synthesis of modified SN38*

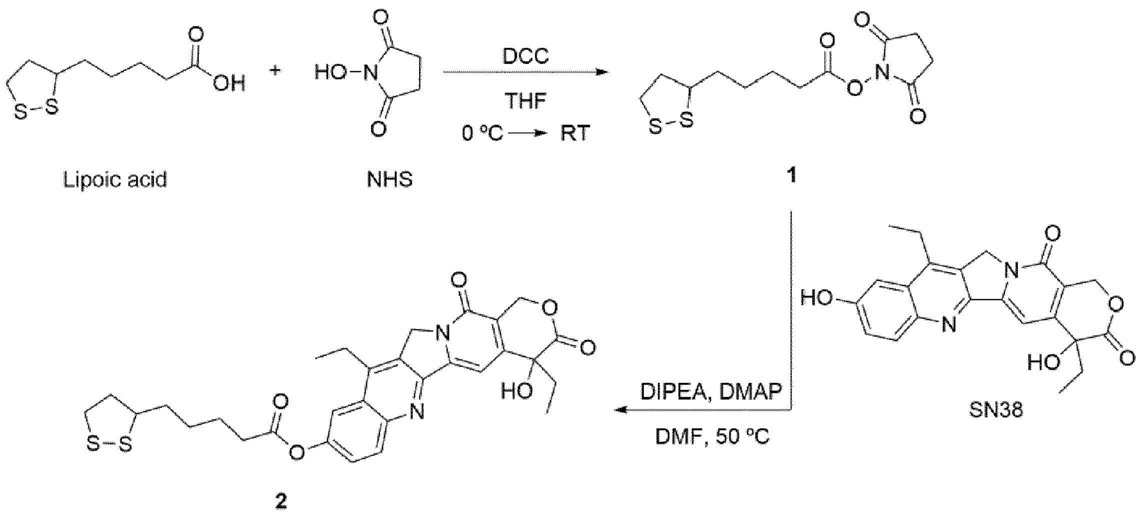


Lipoic acid
NHS
1
SN38
2

Scheme 1: Synthesis of the modified SN38 with the linker for the conjugation with AuNPs. Lipoic acid was activated and incubated with SN38 to yield the desired product.

All reactions were monitored by thin-layer chromatography (TLC), which was performed on sheets of silica gel 60 F254. The TLC analyses were realized with an ultraviolet lamp (254 and 356 nm).

The products were purified by flash column chromatography using Silica Gel (60 Å, 230 x 400 mesh). All NMR spectra were recorded on a Bruker instrument (MHz indicated in brackets) as solutions in CDC13, and the chemical shifts are reported in parts per million (ppm). Coupling constants are reported in hertz (Hz). 2,5-dioxopyrrolidin-1-yl(R)-5-(1,2-dithiolan-3-yl)pentanoate (1):

Lipoic acid (1 g, 1 eq.) and NHS (667 mg, 1.2 eq.) were dissolved in THF (20.8 mL), the solution was stirred at 0 °C for 10 min. A solution of DCC (1.2 g, 1.2 eq.) in THF (1.7 mL) was added slowly to the lipoic acid and NHS solution. The reaction was stirred at room temperature for 5 h. The mixture was filtered, and the solution was kept in the freezer overnight, it was filtered again and the solvent was eliminated in vacuum. The compound 1 was obtained as yellow oil (1.43 g, 99% yield). 1H NMR (400 MHz, CDC13): $\delta$ = 3.60 - 3.50 (m, 4H), 3.20 - 3.06 (m, 1H), 2.81 (s, 4H), 2.61 (t, J = 7.4 Hz, 2H), 2.50- 2.36 (m, 1H), 1.94 - 1.88 (m, 1H), 1.82 - 1.72 (m, 2H), 1.72 - 1.66 (m, 2H), 1.62 - 1.46 (m, 2H). 13C NMR (101 MHz, CDC13): $\delta$= 169.13, 168.42, 67.42, 40.15, 38.52, 34.42, 33.21, 30.79, 25.59, 22.59, 24.36, 23.39.

4,11-diethyl-4-hydroxy-3,14-dioxo-3,4,12,14-tetrahydro-1H-pyrano[3',4':6,7]indolizino[1,2-b]quinolin-9-yl 5-((R)-1,2-dithiolan-3-yl)pentanoate (2):

Compound 1 (56 mg, 2 eq.), SN38 (36 mg, 1 eq.) and DMAP (3 mg) were dissolved in DMF (3.7 mL), then, DIPEA was added to the solution. The reaction was stirred at 50 °C for 18 h. The solvent was eliminated in vacuum and the crude was purified by flash chromatography ($CH_2Cl_2 \rightarrow CH_2Cl_2$/MeOH 40:1) to obtain the compound 2 as white-yellow solid (21 mg, 40 % yield). 1H-NMR (400 MHz, CDC13): $\delta$ = 8.24 (d, J = 9.2 Hz, 1H), 7.83 (d, J = 2.5 Hz, 1H), 7.65 (s, 1H), 7.55 (dd, J = 9.2, 2.5 Hz, 1H), 5.76 (d, J = 16.3 Hz, 1H), 5.35 - 5.29 (m, 1H), 5.26 (s, 2H), 3.68 - 3.59 (m, 1H), 3.25 - 3.10 (m, 4H), 2.69 (t, J = 7.4 Hz, 2H), 2.55 - 2.46 (m, 1H), 2.00 - 1.93 (m, 1H), 1.90 - 1.83 (m, 3H), 1.83 - 1.76 (m, 2H), 1.70 - 1.59 (m, 3H), 1.40 (t, J = 7.7 Hz, 3H), 1.04 (t, J = 7.4 Hz, 3H). 13C-NMR (101 MHz, CDC13): $\delta$= 173.95, 171.85, 157.67, 151.94, 150.18, 149.64, 147.49, 146.95, 145.27, 132.13, 127.48, 127.27, 125.41, 118.55, 114.59, 97.98, 72.76, 66.38, 56.34, 49.40, 40.29, 38.55, 34.61, 34.21, 31.62, 28.75, 24.58, 23.19, 14.01, 7.83.

*2.8 Statistical analysis*

The statistical analysis was performed on the R 3.2.5 program. Anova test for one factor is used for the mean comparison (each condition vs untreated control). Significant differences between mean were accepted when the p-value was lower than: 0.05 (*), 0.01 (**), 0.001 (***).

*Example 1-DNA-based miRNA mimics*

DNA-based miRNA mimics 9,10,11, and 12 (Table 2) were transfected individually into Mel 202 cells and the viability assessed through the alamarBlue assay after 48h. The reduction in cell viability was negligible in all cases (Figure 1a). Then, the inventors evaluated the effect of the miRNA mimics when combined, first in pairs (Figure 1b) and later all together in DNA mix 1 (Figure 1c). Interestingly, the inventors observed 30 % reduction in viability in the last case.

In addition, the inventors studied the effect of the mixture in the expression of c-Met using immunofluorescence (Figure 1d-e). Remarkably, the images revealed significant changes in fluorescence when the cells were treated with the DNA mix, precisely a 67 % reduction in the expression.

*Example 2 Combination therapy*

Since the DNA mix 1 provided a cytotoxic effect and reduces the c-Met expression the inventors decided to study the effect in combination with the chemotherapeutic drug SN38.

The inventors first assessed the IC50 of SN38 (100 nM) in Mel 202 cells (Figure 2a), and then the combined effect of the SN38 with DNA mix 1 (Figure 2b). The inventors observed a significant reduction in the cell viability (50%) using low concentrations of the drug (25 nM) and the DNA mix 1. Remarkably, the effect observed was synergistic according to the method described by Valeriote and Lin [46]. In this method, the efficacy of the individual drugs ($\varepsilon$A, $\varepsilon$B) is compared to the combined treatment ($\varepsilon$A+B). It is synergic if $\varepsilon A+B < (\varepsilon A \times \varepsilon B)/100$, additive, if $\varepsilon A+B = (\varepsilon A \times \varepsilon B)/100$, sub-additive if $(\varepsilon A \times \varepsilon B)/100 < 327\ \varepsilon A+B < \varepsilon A$, provided $\varepsilon A < \varepsilon B$, interference, if $\varepsilon A < \varepsilon A+B < \varepsilon B$, when $\varepsilon A < \varepsilon B$, and antagonistic, if $\varepsilon B < \varepsilon A+B$, when $\varepsilon A < \varepsilon B$. According to this method, our treatment had a synergic effect.

This result highlights the potential of this approach, where reprograming the behavior of UM by oligonucleotides allows a significant reduction of the dose of SN38 to 25 nM and, therefore, it could contribute to the decrease of side effects. What is more, the reduction of c-Met expression observed in the combination approach should lead to a reduction of drug resistance, as mentioned previously

After that, the inventors study the effect of this approach on the cell cycle (Figure 3). Cells were harvested after treatment and analyzed by flow cytometry. The effect of the DNA mix 1 was negligible compared with the effect obtained with SN38, which significantly increases G2/M.

*Example 3- Gold nanoparticles as carriers*

AuNPs were prepared using the Turkevich method, and modified with the oligonucleotides (DNA mix and siRNA mix) and SN38 as described in the previous section. Particularly, the bare nanoparticles were incubated with oligonucleotides (DNA mix 2 or RNA mix) modified with a thiol moiety in the presence of NaCl for 16 h. In the case of the nanoparticle modified with SN38 the particles were previously incubated with the aptamer AS1411 for 30 min and then the modified SN38 (2) was added to the solution (Scheme 2).

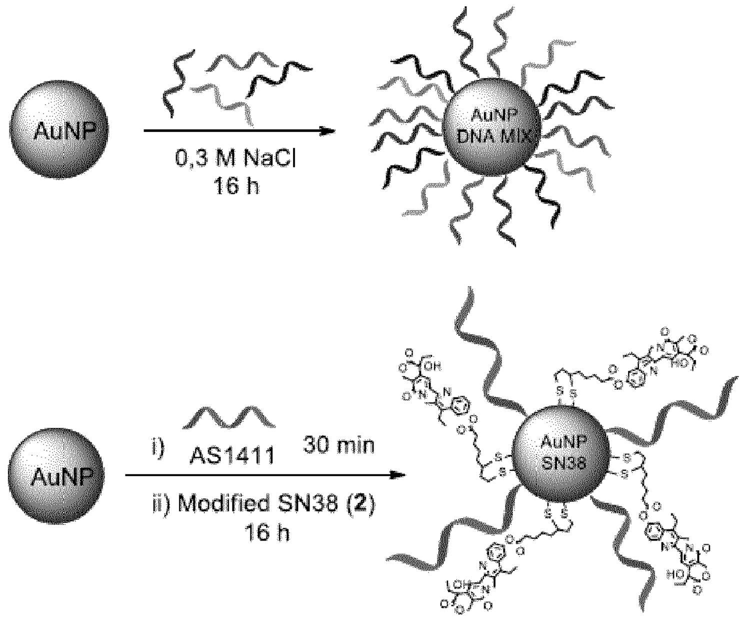


Scheme 2: Functionalized process of AuNP DNA MIX and AuNP SN38

Once the unbound material was removed the sizes of the three formulations (AuNPs DNA mix, AuNPs siRNA and AuNP SN38 mix) was studied by DLS, where the average size observed was 13-15 nm (Figure 4a-c). The UV-vis spectrum revealed the characteristic plasmond band at 520 of AuNPs (Figure 4d).

The activity of functionalize AuNPs was evaluated in Mel 202 cells, where the AuNP DNA mix did not reduce the viability of the cells. When the AuNP SN38 was added the viability was reduced, and this reduction was even higher when used in combination with AuNP DNA mix (Figure 5a).

The inventors assessed the effect when siRNAs were used instead of DNA in the nanoparticles, and the inventors observed a reduction of the viability, which was increased when combined with AuNP SN38. (Figure 5b) In this case, the inhibition of AuNP siRNA was higher than AuNP DNA, and it was even higher when combined with SN38. Interestingly, in both cases, the effect was synergistic probably due to their different mechanism of action and the different process and pathways involved in both therapeutics.

The cell cycle was affected when SN38 was present, but not when siRNAs were used in the formulation (Figure 6).

## Claims

1. A combination comprising a miRNA-34a mimic, a miRNA-182 mimic, a miRNA-137 mimic and a miRNA-144 mimic.

2. The combination according to claim 1 wherein the miRNA-34a mimic, the miRNA-182 mimic, the miRNA-137 and the miRNA-144 mimic are independently selected from the group consisting of:

   (i) a DNA oligonucleotide comprising a DNA version of the sequence of the mature miRNA or a functionally variant equivalent thereof,
   (ii) a double stranded RNA, wherein the sequence of one of the strands comprises the sequence of the mature

miRNA or a functionally variant equivalent thereof and
(iii) a polynucleotide encoding the miRNA.

3. The combination according to claim 2 wherein the miRNA mimics are DNA antisense oligonucleotides and wherein the miRNA-34a mimic comprises the DNA version of the sequence of the mature miRNA-34a as defined in the miRbase database under accession number MIMAT0000255 or a functionally variant equivalent thereof, the miRNA-182 mimic comprises the DNA version of the sequence of the mature miRNA-182 as defined in the miRbase database under accession number MIMAT0000259 or a functionally variant equivalent thereof, the miRNA-137 mimic comprises the DNA version of the sequence of the mature miRNA-137 as defined in the miRbase database under accession number MIMAT0000429 or a functionally variant equivalent thereof and/or wherein the miRNA-144 mimic comprises the DNA version of the sequence of the mature miRNA-144 as defined in the miRbase database under accession number MIMAT0004600 or a functionally variant equivalent thereof.

4. The combination according to claim 3, wherein the miRNA-34a mimic comprises a sequence shown in SEQ ID NO: 5, SEQ ID NO:9 or a functionally variant equivalent thereof, the miRNA-182 mimic comprises a sequence shown in SEQ ID NO: 6, SEQ ID NO:10 or a functionally variant equivalent thereof, the miRNA-137 mimic comprises a sequence shown in SEQ ID NO: 7, SEQ ID NO:11 or a functionally variant equivalent thereof and wherein the miRNA-144 mimic comprises a sequence shown in SEQ ID NO: 8, SEQ ID NO:12 or a functionally variant equivalent thereof.

5. The combination according to claim 2 wherein the miRNA mimics are double stranded inhibitory RNAs comprising two anticomplementary RNA sequence regions and wherein the miRNA34a mimic comprises a RNA sequence region corresponding to the mature miRNA-34a as defined in the miRbase database under accession number MIMAT0000255 or a functionally variant equivalent thereof, wherein the miRNA-182 mimic comprises a RNA sequence region corresponding to the mature miRNA-182 as defined in the miRbase database under accession number MIMAT0000259 or a functionally variant equivalent thereof, the miRNA-137 mimic comprises a RNA sequence region corresponding to the mature miRNA-137 as defined in the miRbase database under accession number MIMAT0000429 or a functionally variant equivalent thereof and/or wherein the miRNA-144 mimic comprises a RNA sequence region corresponding to the mature miRNA-144 as defined in the miRbase database under accession number MIMAT0004600 or a functionally variant equivalent thereof.

6. The combination according to claim 5 wherein the miRNA34a mimic comprises a first sequence region comprising the sequence shown in SEQ ID NO: 17 or a functionally variant equivalent thereof and a second sequence region comprising the sequence shown in SEQ ID NO: 18 or a functionally variant equivalent thereof, the miRNA-182 mimic comprises a first sequence region comprising the sequence shown in SEQ ID NO: 19 or a functionally variant equivalent thereof and a second sequence region comprising the sequence shown in SEQ ID NO: 20 or a functionally variant equivalent thereof, the miRNA-137 mimic comprises a first sequence region comprising the sequence shown in SEQ ID NO: 21 or a functionally variant equivalent thereof and a second sequence region comprising the sequence shown in SEQ ID NO: 22 or a functionally variant equivalent thereof and wherein the miRNA-144 mimic comprises a first sequence region comprising the sequence shown in SEQ ID NO: 23 or a functionally variant equivalent thereof and a second sequence region comprising the sequence shown in SEQ ID NO: 24 or a functionally variant equivalent thereof.

7. The combination according to any of claim 1 to 6 further comprising a chemotherapeutic compound.

8. The combination according to claim 7 wherein the chemotherapeutic compound is a topoisomerase inhibitor.

9. The combination according to any of claims 1 to 8, wherein the miRNA mimics are covalently coupled to a nanoparticle.

10. The combination according to claim 9 wherein the miRNA mimics are covalently coupled to the nanoparticle by a disulfide bond and wherein if the miRNA mimics comprises the sequences SEQ ID NO: 17, 19, 21 or 23 or a functionally variant equivalent thereof the disulfide bond is formed at the 5'end of said sequences and wherein if the miRNA mimics comprises the sequences SEQ ID NO: 9 to 12 or a functionally variant equivalent thereof the disulfide bond is formed at the 3' end of said sequences.

11. The combination according to claims 9 or 10 wherein the chemotherapeutic compound present in a nanoparticle is functionalized with an oligonucleotide having the sequence shown in SEQ ID NO: 25, wherein said sequence is covalently bond to the nanoparticle by a disulfide bond, said disulfide bond present at the 3' end of said sequence.

**12.** The combination according to any of claims 1 to 11 for use in medicine.

**13.** The combination according to any of claims 1 to 11 for use in the treatment and/or prevention of cancer.

**14.** The combination for use according to claim 13 wherein the cancer is uveal melanoma.

**15.** The combination according to any of claims 1 to 6, 9 or 10 for use as an adjuvant in the treatment of cancer with a chemotherapeutic compound.

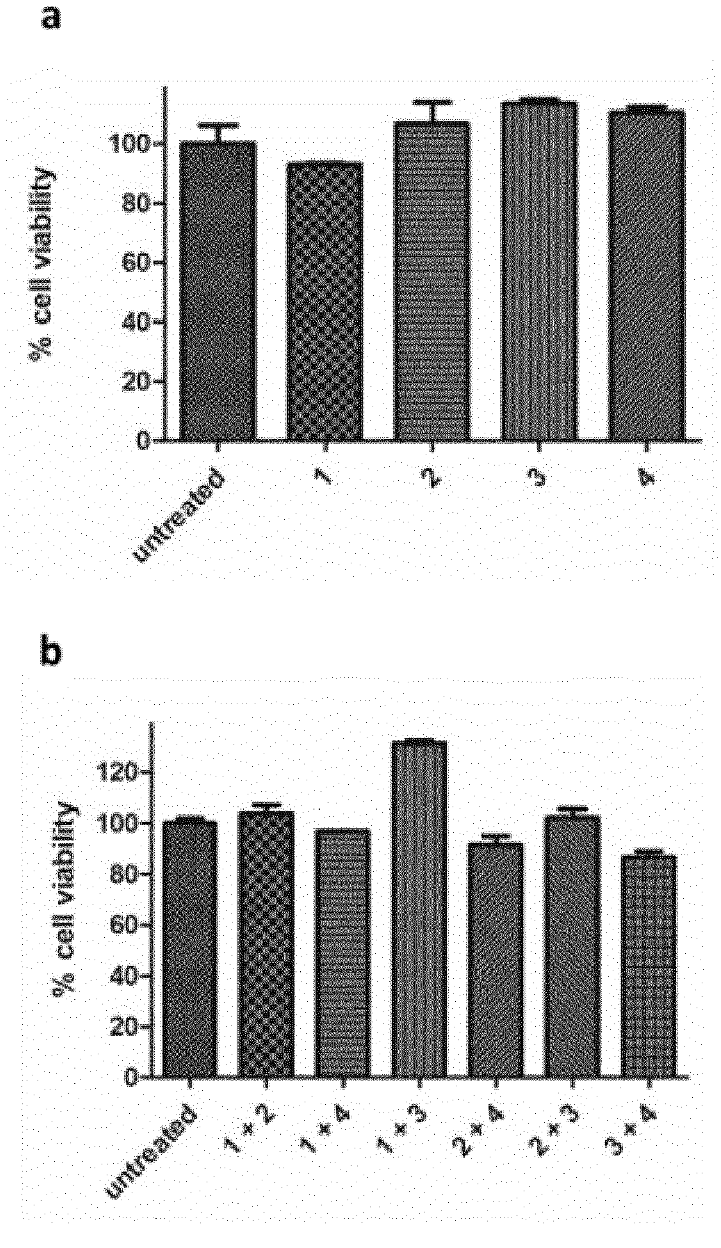
a
% cell viability
0
20
40
60
80
100
untreated
1
2
3
4
b
% cell viability
0
20
40
60
80
100
120
untreated
1 + 2
1 + 4
1 + 3
2 + 4
2 + 3
3 + 4

Fig. 1

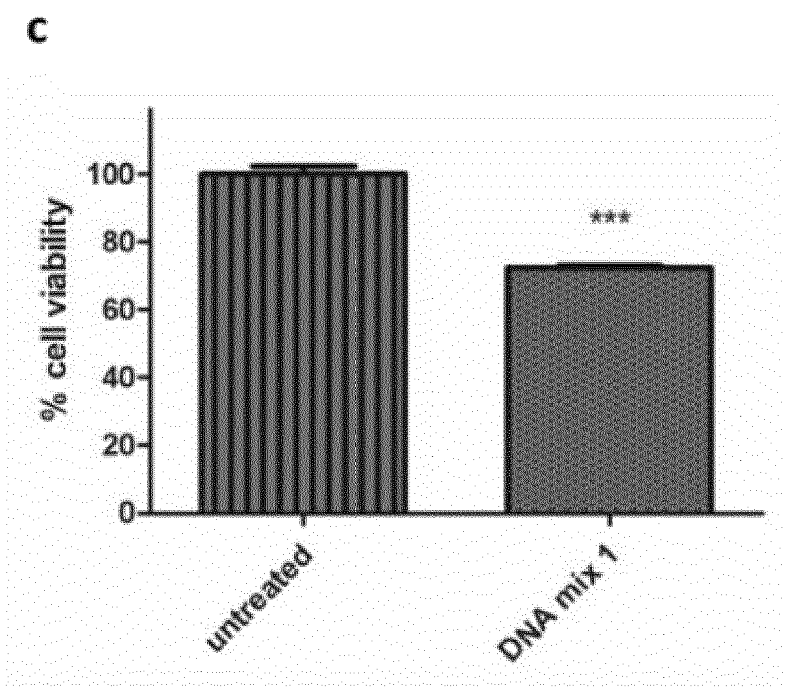
c
% cell viability
100
80
60
40
20
0
***
untreated
DNA mix 1

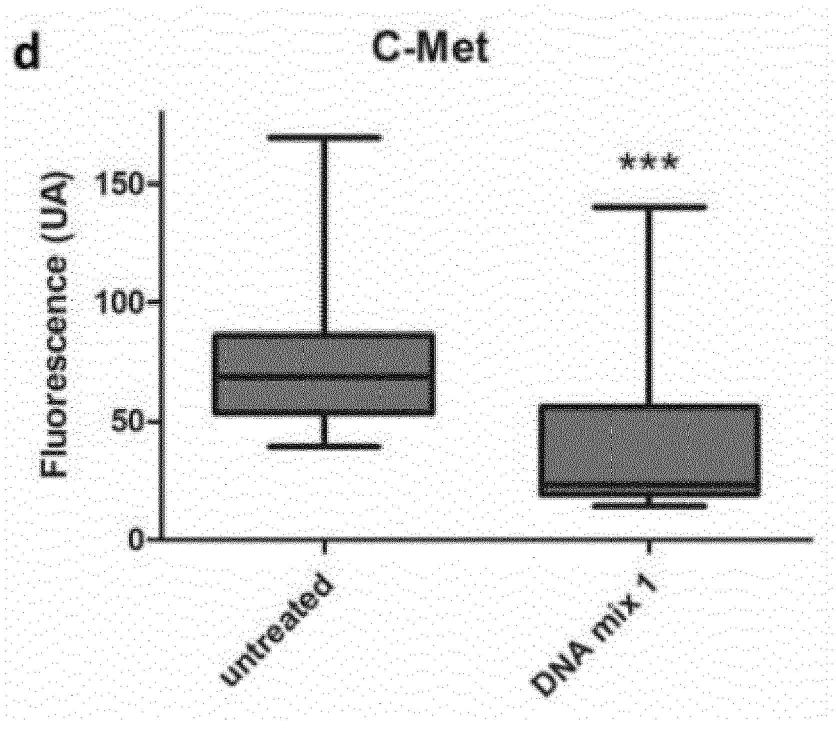
d
C-Met
Fluorescence (UA)
150
100
50
0
***
untreated
DNA mix 1

Fig. 1 (Cont.)

e

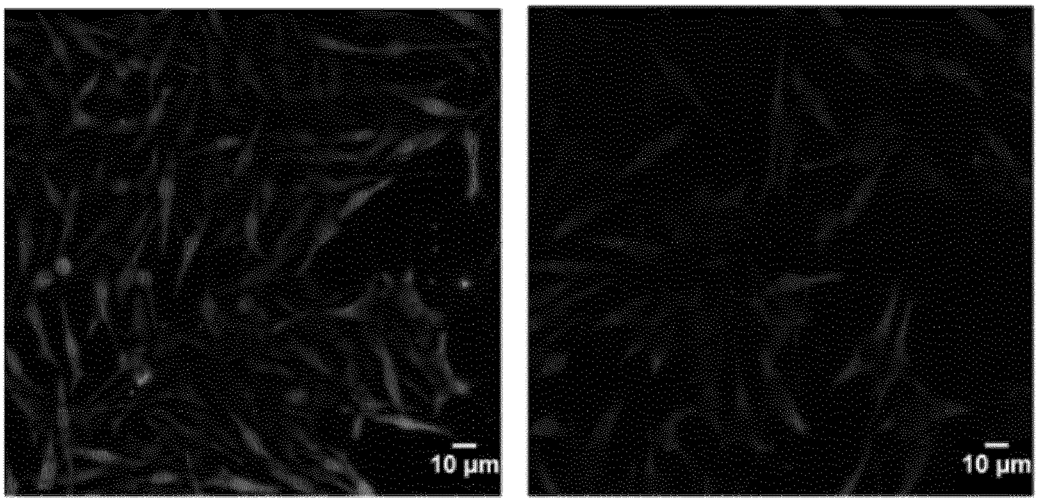


Fig. 1 (Cont.)

a

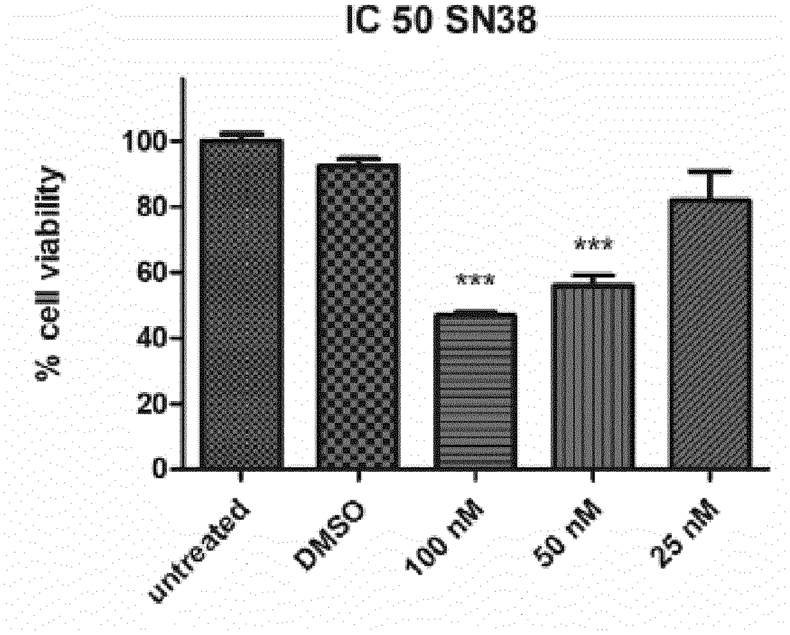
IC 50 SN38
% cell viability
0
20
40
60
80
100
***
***
untreated
DMSO
100 nM
50 nM
25 nM

b

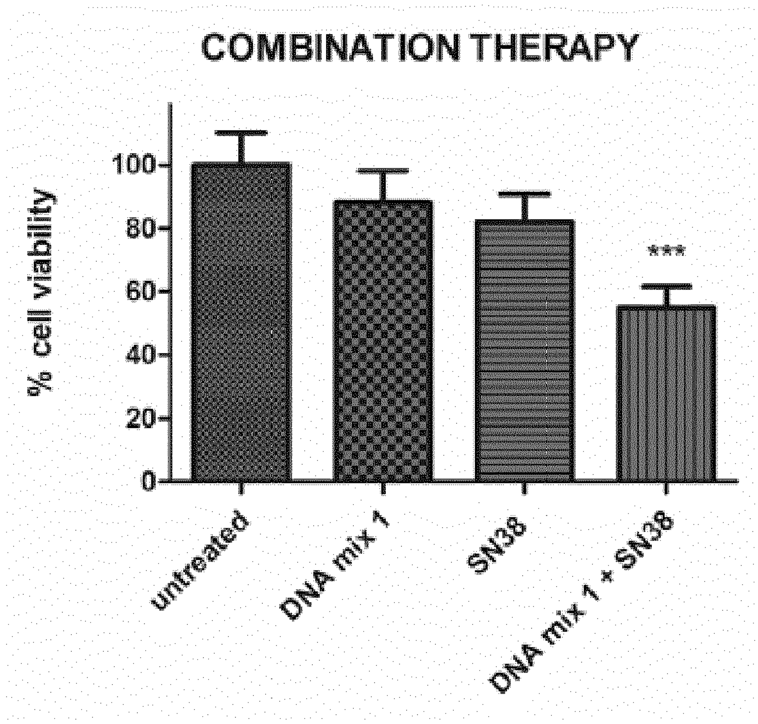
COMBINATION THERAPY
% cell viability
0
20
40
60
80
100
***
untreated
DNA mix 1
SN38
DNA mix 1 + SN38

Fig. 2

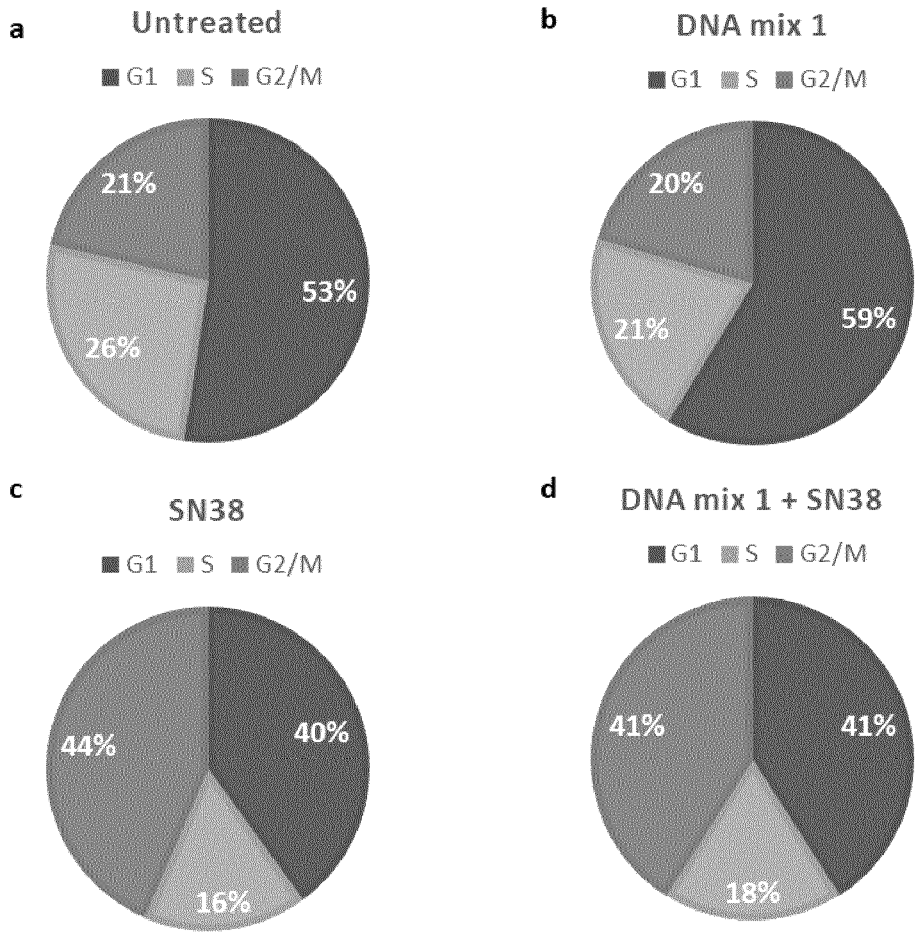
a
Untreated
G1 S G2/M
21%
53%
26%
b
DNA mix 1
G1 S G2/M
20%
59%
21%
c
SN38
G1 S G2/M
44%
40%
16%
d
DNA mix 1 + SN38
G1 S G2/M
41%
41%
18%

Fig. 3

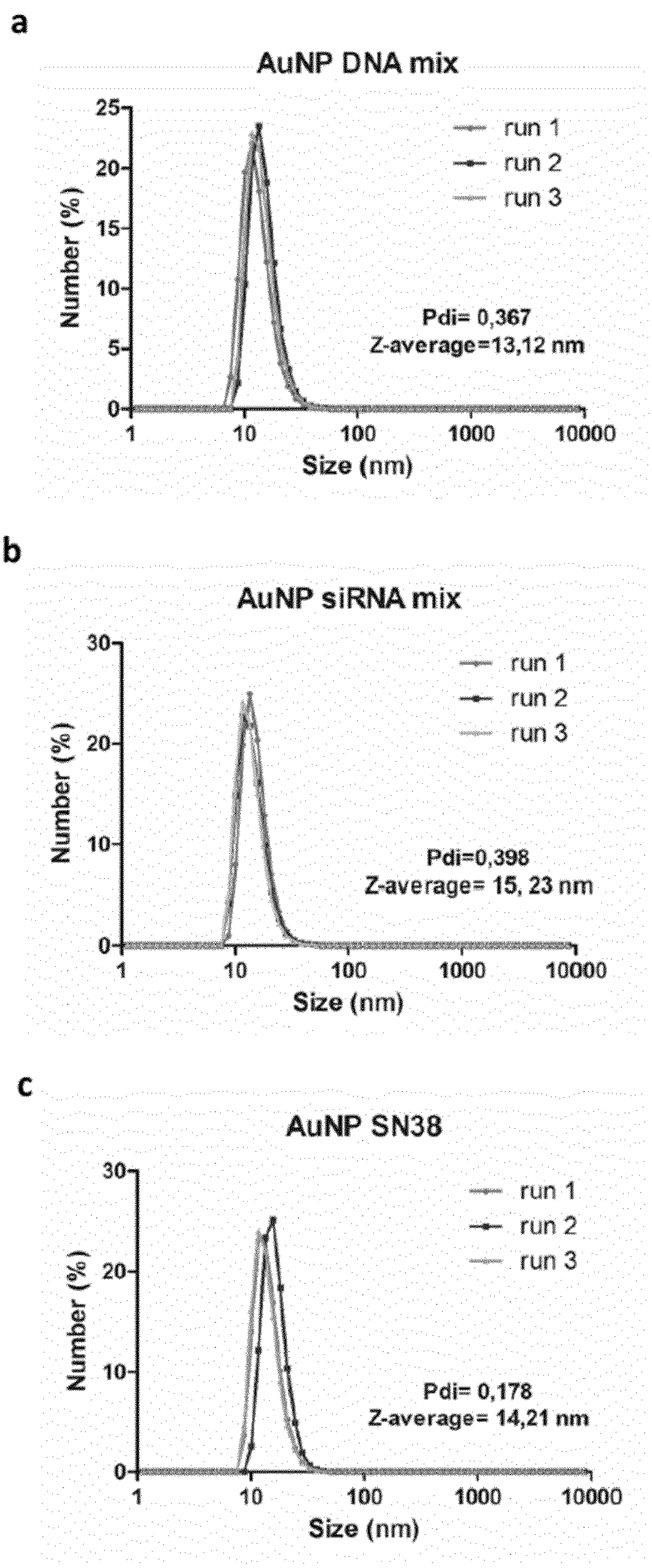
a
AuNP DNA mix
run 1
run 2
run 3
Number (%)
Pdi= 0,367
Z-average=13,12 nm
Size (nm)
b
AuNP siRNA mix
run 1
run 2
run 3
Number (%)
Pdi=0,398
Z-average= 15, 23 nm
Size (nm)
c
AuNP SN38
run 1
run 2
run 3
Number (%)
Pdi= 0,178
Z-average= 14,21 nm
Size (nm)

Fig. 4

d

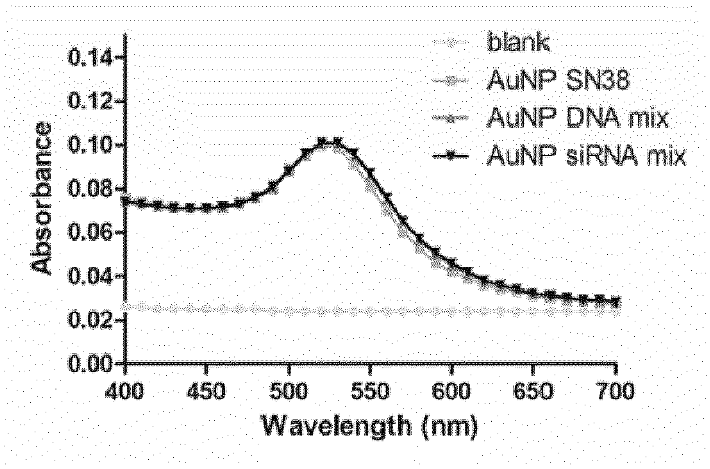
0.14
0.12
0.10
0.08
0.06
0.04
0.02
0.00
Absorbance
400
450
500
550
600
650
700
Wavelength (nm)
blank
AuNP SN38
AuNP DNA mix
AuNP siRNA mix

Fig. 4 (Cont.)

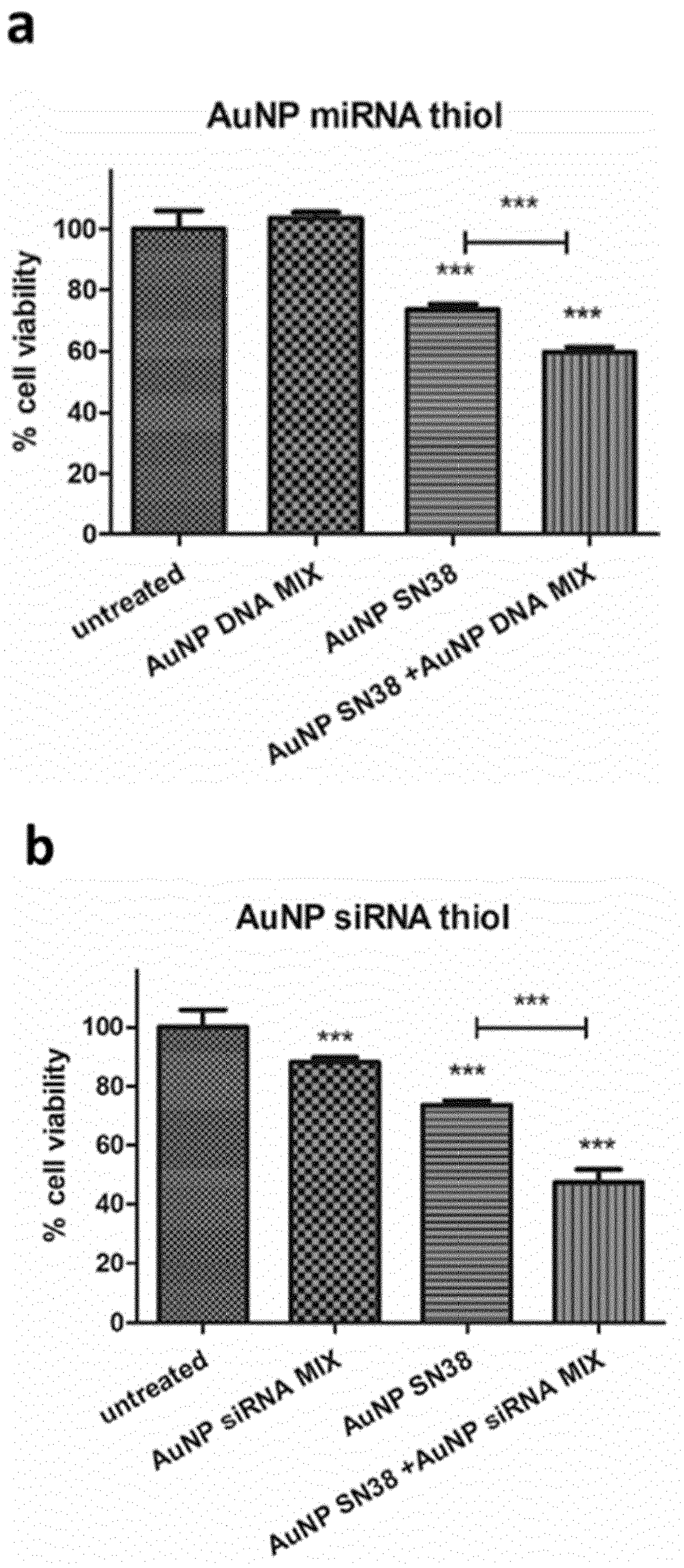
a
AuNP miRNA thiol
% cell viability
0
20
40
60
80
100
***
***
***
untreated
AuNP DNA MIX
AuNP SN38
AuNP SN38 +AuNP DNA MIX
b
AuNP siRNA thiol
% cell viability
0
20
40
60
80
100
***
***
***
***
untreated
AuNP siRNA MIX
AuNP SN38
AuNP SN38 +AuNP siRNA MIX

Fig. 5

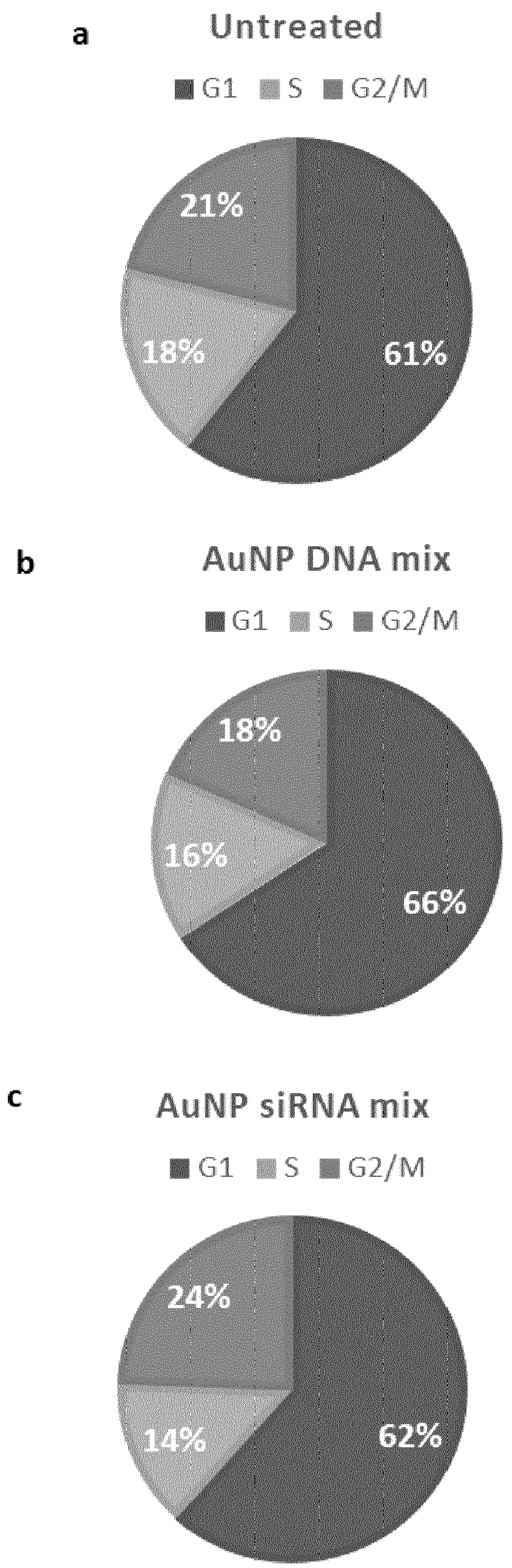
a
Untreated
G1
S
G2/M
21%
18%
61%
b
AuNP DNA mix
G1
S
G2/M
18%
16%
66%
c
AuNP siRNA mix
G1
S
G2/M
24%
14%
62%

Fig. 6

d

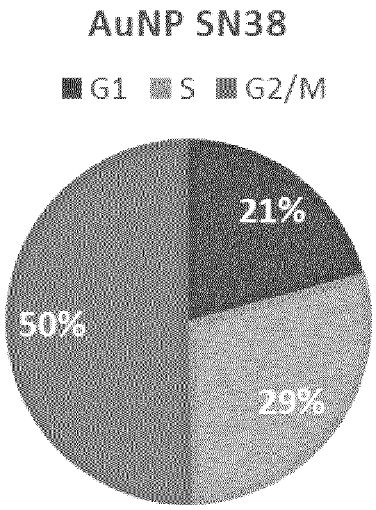
AuNP SN38
G1
S
G2/M
21%
29%
50%

e

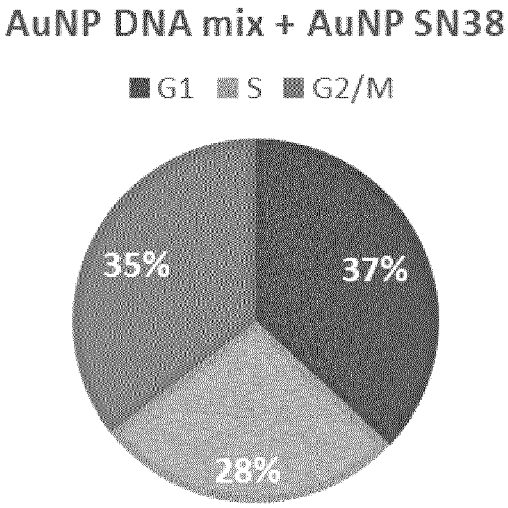
AuNP DNA mix + AuNP SN38
G1
S
G2/M
37%
28%
35%

Fig. 6 (Cont.)

Europäisches Patentamt
European Patent Office
Office européen des brevets

# EUROPEAN SEARCH REPORT

Application Number
EP 18 38 2676

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YONGYUN LI ET AL: "Role of Epigenetics in Uveal Melanoma", INTERNATIONAL JOURNAL OF BIOLOGICAL SCIENCES, vol. 13, no. 4, 1 January 2017 (2017-01-01), pages 426-433, XP055571170, Australia ISSN: 1449-2288, DOI: 10.7150/ijbs.18331 * table 1 * ----- | 1-15 | INV. C12N15/113 |
| A | ZHENG LI ET AL: "MicroRNA dysregulation in uveal melanoma: a new player enters the game", ONCOTARGET, vol. 6, no. 7, 10 March 2015 (2015-03-10), XP055571189, DOI: 10.18632/oncotarget.2923 * the whole document * ----- | 1-15 | |
| A | BEATRIZ ÁLVAREZ-RODRÍGUEZ ET AL: "Recent advances in uveal melanoma treatment", MEDICINAL RESEARCH REVIEWS, vol. 37, no. 6, 1 November 2017 (2017-11-01), pages 1350-1372, XP055571672, US ISSN: 0198-6325, DOI: 10.1002/med.21460 * table 2 * ----- | 1-15 | TECHNICAL FIELDS SEARCHED (IPC) C12N |
| A | WO 2015/085164 A2 (CLEVELAND CLINIC FOUNDATION [US]) 11 June 2015 (2015-06-11) * the whole document * ----- -/-- | 1-15 | |

The present search report has been drawn up for all claims

1

| Place of search | Date of completion of the search | Examiner |
|---|---|---|
| The Hague | 20 March 2019 | Romano, Alper |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 18 38 2676

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | PAULA MILÁN ROIS ET AL: "Reprogramming Cells for Synergistic Combination Therapy with Nanotherapeutics against Uveal Melanoma", BIOMIMETICS, vol. 3, no. 4, 25 September 2018 (2018-09-25), page 28, XP055571699, DOI: 10.3390/biomimetics3040028 * the whole document * ----- | | |
| | | | TECHNICAL FIELDS SEARCHED (IPC) |

1

The present search report has been drawn up for all claims

| Place of search | Date of completion of the search | Examiner |
|---|---|---|
| The Hague | 20 March 2019 | Romano, Alper |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 18 38 2676

This annex lists the patent family members relating to the patent documents cited in the above-mentioned European search report.
The members are as contained in the European Patent Office EDP file on
The European Patent Office is in no way liable for these particulars which are merely given for the purpose of information.

20-03-2019

| Patent document cited in search report | Publication date | Patent family member(s) | Publication date |
|---|---|---|---|
| WO 2015085164 A2 | 11-06-2015 | US 2015159225 A1<br>WO 2015085164 A2 | 11-06-2015<br>11-06-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006135436 A
- WO 2006012221 A
- US 5350674 A
- US 5585362 A
- US 5328470 A


### Non-patent literature cited in the description


- **BENTON ; DAVIS.** *Science,* 1977, vol. 196, 180
- **GRUNSTEIN ; HOGNESS.** *Proc. Natl. Acad. Sci., USA,* 1975, vol. 72, 3961
- **AUSUBEL et al.** Current Protocols in Molecular Biology. Wiley Interscience, 2001
- **BERGER ; KIMMEL.** Guide to Molecular Cloning Techniques. Academic Press, 1987
- **SAMBROOK et al.** Molecular Cloning: A Laboratory Manual. Cold Spring Harbor Laboratory Press
- **QUE-GEWIRTH NS.** *Gene Ther.,* February 2007, vol. 14 (4), 283-91
- **ONEY S.** Fall. *Oligonucleotides,* 2007, vol. 17 (3), 265-74
- **CHEN, X et al.** *Nucleic Acids Res.,* 2006, 34
- **R. ELGHANIAN ; J.J. STORHOFF ; R.C. MUCIC ; R.L. LETSINGER ; C.A. MIRKIN.** Selective colorimetric detection of polynucleotides based on the distance-dependent optical properties of gold nanoparticles. *Science,* 1997, vol. 277, 1078-1081
- **MUCIC et al.** *Chem. Commun.,* 1996, 555-557
- **Z. LI ; R.C. JIN ; C.A. MIRKIN ; R.L. LETSINGER.** Multiple thiol-anchor capped DNA-gold nanoparticle conjugates. *Nucleic Acids Res.,* 2002, vol. 30, 1558-1562
- Remington's Pharmaceutical Sciences. Mack Publication Co, 1991
- **SAMBROOK et al.** MOLECULAR CLONING: A LABORATORY MANUAL. Cold Spring Harbor Press, 2001, vol. 1-3
- **GHOSH et al.** *Glycobiology,* 1991, vol. 5, 505-10
- **CHEN et al.** *PNAS,* 1994, vol. 91, 3054-3057
- **LATORRE, A. et al.** *Nanoscale,* 2014, vol. 6, 7436
- **POSCH, C. ; LATORRE, A. ; CROSBY, M. B. ; CELLI, A. ; LATORRE, A. ; VUJIC, I. ; SANLORENZO, M. ; GREEN, G. A. ; WEIER, 479 J. ; ZEKHTSER, M.** *S. Biomed. Microdevices,* 2015, vol. 17, 15
- **RIEMER, J. ; HOEPKEN, H. H. ; CZERWINSKA, H. ; ROBINSON, S. R. ; DRINGEN, R.** *Anal. Biochem.,* 2004, vol. 331, 370-375
- **DEL MORAL, Á.** *Cell Fluorescence Weber, https://github.com/DelmoPy/Cell-Fluorescence-Weber*